(19) Europäisches Patentamt
European Patent Office
Office européen des brevets

(11) **EP 1 414 823 B1**

(12) **FASCICULE DE BREVET EUROPEEN**

(45) Date de publication et mention de la délivrance du brevet:
**02.05.2007 Bulletin 2007/18**

(21) Numéro de dépôt: **02770041.8**

(22) Date de dépôt: **19.07.2002**

(51) Int Cl.:
***C07D 487/22*** (2006.01) ***B01D 53/86*** (2006.01)

(86) Numéro de dépôt international:
**PCT/FR2002/002591**

(87) Numéro de publication internationale:
**WO 2003/011865 (13.02.2003 Gazette 2003/07)**

(54) **MATERIAUX POUR LE PIEGEAGE SELECTIF DU MONOXYDE DE CARBONE**

MATERIAL ZUR SELEKTIVEN TRENNUNG VON KOHLENMONOXID

MATERIALS FOR SELECTIVE TRAPPING OF CARBON MONOXIDE

(84) Etats contractants désignés:
**AT BE BG CH CY CZ DE DK EE ES FI FR GB GR IE IT LI LU MC NL PT SE SK TR**

(30) Priorité: **01.08.2001 FR 0110317**

(43) Date de publication de la demande:
**06.05.2004 Bulletin 2004/19**

(73) Titulaire: **L'AIR LIQUIDE, Société Anonyme pour l'Etude et l'Exploitation des Procédés Georges Claude**
**75321 Paris Cedex 07 (FR)**

(72) Inventeurs:
- **JEROME, François**
  **F-86000 POITIERS (FR)**
- **DUBOIS, Géraud**
  **Palo Alto, CA 94306 (US)**
- **BRANDES, Stéphane**
  **F-21100 Dijon (FR)**
- **CANARD, Gabriel**
  **F-21000 Dijon (FR)**
- **BARBE, Jean-Michel**
  **F-21490 Bretigny (FR)**
- **GUILARD, Roger**
  **F-21121 Dijon (FR)**
- **ROUX-FOUILLET, Bruno**
  **F-60300 SENLIS (FR)**
- **LEDON, Henry**
  **F-78000 Versailles (FR)**

(74) Mandataire: **Conan, Philippe Claude et al**
**L'Air Liquide**
**Direction de la Propriété Industrielle**
**75 Quai d'Orsay**
**75321 Paris Cedex 07 (FR)**

(56) Documents cités:
**WO-A-00/18771** **US-A- 4 970 188**

- **GRYKO, DANIEL T. ET AL: "A Simple and Versatile One-Pot Synthesis of meso-Substituted trans-A2B-Corroles" JOURNAL OF ORGANIC CHEMISTRY (2001), 66(12), 4267-4275, XP002197037**
- **GOLUBKOV, GALINA ET AL: "High-valent manganese corroles and the first perhalogenated metallocorrole catalyst" ANGEWANDTE CHEMIE, INTERNATIONAL EDITION (2001), 40 (11), 2132-2134, XP001053950**
- **SIMKHOVICH, LILIYA ET AL: "Synthesis and characterization of germanium, tin, phosphorus, iron, and rhodium complexes of tris (pentafluorophenyl)corrole, and the utilization of the iron and rhodium corroles as cyclopropanation catalysts" CHEMISTRY--A EUROPEAN JOURNAL (2001), 7(5), 1041-1055, XP002197039**
- **PAOLESSE, ROBERTO ET AL: "Synthesis and Functionalization of meso-Aryl-Substituted Corroles" JOURNAL OF ORGANIC CHEMISTRY (2001), 66(2), 550-556, XP002197040**
- **BRIÑAS ET AL.: "Triarylcorroles by oxidative coupling of triaryltetrapyrranes" SYNLETT., no. 3, 2000, pages 442-444, XP002197514 THIEME VERLAG, STUTTGART., DE ISSN: 0936-5214**
- **N.S. GENOKHOVA ET AL.: "Change in the coordination number of the cobalt atom in octadehydrocorrin complexes" JOURNAL OF GENERAL CHEMISTRY USSR., vol. 49, no. 7(2), 1980, pages 1626-29, XP002197515 CONSULTANTS BUREAU. NEW YORK., US**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

- GRYKO, DANIEL T.: "A simple, rational synthesis of meso-substituted A2B-corroles" CHEMICAL COMMUNICATIONS (CAMBRIDGE) (2000), (22), 2243-2244, XP002197042

## Description

L'invention a pour objet de nouveaux composés capables de complexer le monoxyde de carbone et leur utilisation dans les détecteurs dudit gaz.

Les oxydes métalliques semi-conducteurs tels que ZnO, $TiO_2$, $SnO_2$, $WO_3$, $Ga_2O_3$ sont très largement utilisés comme supports actifs de détecteurs de monoxyde de carbone. La caractéristique essentielle de ces composés est leur sensibilité importante pour le monoxyde de carbone mais leur température d'utilisation est élevée (= 350°C).

D'autres oxydes métalliques, ceux ayant une structure pérovskite ($ABO_3$ où A et B sont des métaux différents) ont été essayés pour détecter le monoxyde de carbone. Ces composés ont une bonne sensibilité pour ce gaz avec des températures d'utilisation moins élevées, de l'ordre de 150-200°C.

L'hémoglobine, la myoglobine et leurs modèles sont capables de coordonner l'oxygène et le monoxyde de carbone en solution à température ambiante. Cette coordination intervient au niveau de l'atome de fer(II) de l'hémoprotéine. Pour ce type de composé, la sélectivité pour l'un ou l'autre des gaz est donnée par M qui est le rapport des pressions partielles à demi - saturation des deux gaz[1].

$$M = P_{1/2}O_2/P_{1/2}CO$$

Une valeur importante de M traduit une plus grande affinité pour le monoxyde de carbone que pour l'oxygène.

La myoglobine, pour laquelle M est compris entre 20 et 40 et l'hémoglobine, pour laquelle M est environ égal à 150, ont une forte affinité pour l'oxygène à température ambiante.

En revanche, le composé dénommé (T*o*-PivPP)Fe(1,2$Me_2$Im) et représenté par la Figure 1, pour lequel la valeur de M en solution et à température ambiante est de l'ordre de 4 000 avec une pression partielle de demi - saturation de monoxyde de carbone ($P_{1/2}CO$) environ égale à 9 x $10^{-3}$ Torr (1 Torr = 1 mm Hg = $10^5$ Pa. / 760), a plus d'affinité pour le monoxyde de carbone que pour l'oxygène à température ambiante. Cependant, la stabilité à long terme de ce genre de composés est limitée, notamment du fait de l'oxydation du fer(II) en fer(III) à l'air en présence d'humidité, ce qui inhibe ensuite toute coordination ultérieure du monoxyde de carbone.

Au regard des données de la littérature, il existe un besoin de composés, capables de coordonner sélectivement et de façon réversible, le monoxyde de carbone à température ambiante et qui présentent une grande stabilité dans le temps.

Le brevet américain N° 4,970,188 divulgue des composés organométalliques supportés dérivés de phtalocyanines pour éliminer les oxydes d'azote et le monoxyde de carbone, de gaz d'échappement.

Parmi les macrocycles candidats possibles, les inventeurs se sont intéressés au corrole, représenté par la Figure 2. C'est un tétrapyrrole comme les porphyrines[2]. A l'inverse des porphyrines, le corrole possède trois atomes de carbone pontant deux unités pyrroliques au lieu de quatre. Cette contraction de cycle confère des propriétés remarquables aux corroles. En effet, la cavité macrocyclique est de plus petite taille que celle des porphyrines et les trois fonctions amine lui permettent de stabiliser des métaux dans des degrés d'oxydation plus élevés.

C'est pourquoi l'invention a pour objet le procédé tel que défini à la revendication 1.

Par radical alkyle, linéaire ou ramifié, comportant de 1 à 4 atomes de carbone, on désigne un des radicaux méthyle, éthyle, propyle, isopropyle, butyle, 1-méthyl propyle, 2-méthyl propyle ou 1,1-diméthyl éthyle.

Par radical alkyloxy, linéaire ou ramifié, comportant de 1 à 4 atomes de carbone, on désigne un des radicaux méthoxy, éthoxy, propoxy, isopropoxy, butoxy, 1-méthyl propoxy, 2-méthyl propoxy ou 1,1-diméthyl éthoxy.

Par radical phényle substitué par un ou plusieurs groupes identiques ou différents choisis indépendamment les uns des autres parmi les radicaux, vinyle, cyano, carboxy, hydroxy, nitro, amino, bromo, chloro, fluoro, iodo, benzyloxy, les radicaux alkyles linéaires ou ramifiés, comportant de 1 à 4 atomes de carbone ou les radicaux alkyloxy linéaires ou ramifiés, comportant de 1 à 4 atomes de carbone, lesdits radicaux alkyles et alkyloxy étant eux-mêmes soit non substitués, soit substitués par un ou plusieurs groupes, bromo, chloro, fluoro ou iodo, on désigne par exemple, les radicaux 2,3,4,5,6-pentafluoro phényle, 2-fluoro phényle, 2-bromo phényle, 2-cyano phényle, 2-carboxy phényle, 2-chloro phényle, 2-iodo phényle, 2-nitro phényle, 2-méthoxy phényle, 2-hydroxy phényle, 2-amino phényle, 2-hydroxyméthyl phényle, 2-vinyl phényle, 2-méthoxyméthyl phényle, 3-fluoro phényle, 3-bromo phényle, 3-cyano phényle, 3-carboxy phényle, 3-chloro phényle, 3-iodo phényle, 3-nitro phényle, 3-méthoxy phényle, 3-hydroxy phényle, 3-amino phényle, 3-hydroxyméthyl phényle, 3-vinyl phényle, 3-méthoxyméthyl phényle, 4-fluoro phényle, 4-bromo phényle, 4-cyano phényle, 4-carboxy phényle, 4-chloro phényle, 4-iodo phényle, 4-nitro phényle, 4-méthoxy phényle, 4-hydroxy phényle, 2-tolyle, 3-tolyle ou 4-tolyle, 3,4-xylyle, 3,4-diméthoxy phényle, 3,4-dichloro phényle, 4-amino phényle, 4-hydroxyméthyl phényle, 4-vinyl phényle ou 4-méthoxyméthyl phényle.

Selon un premier aspect particulier, l'invention a pour objet un procédé tel que défini ci-dessus pour lequel dans la formule (I), un seul des groupes $R_a$ $R_b$ ou $R_c$ représente un radical phényle non substitué ou un radical phényle

substitué par un ou plusieurs groupes identiques ou différents choisis indépendamment les uns des autres parmi les radicaux, vinyle, hydroxy, nitro, cyano, carboxy, amino, bromo, chloro, fluoro, iodo, benzyloxy, les radicaux alkyles linéaires ou ramifiés, comportant de 1 à 4 atomes de carbone ou les radicaux alkyloxy linéaires ou ramifiés, comportant de 1 à 4 atomes de carbone, lesdits radicaux alkyles et alkyloxy étant eux-mêmes soit non substitués, soit substitués par un ou plusieurs groupes, bromo, chloro, fluoro ou iodo.

Selon un deuxième aspect particulier, l'invention a pour objet un procédé tel que défini ci-dessus pour lequel dans la formule (I), un seul des groupes $R_a$, $R_b$ ou $R_c$, représente un radical choisi parmi les radicaux 4-bromo phényle, 4-chloro phényle, 4-iodo phényle, 4-nitro phényle, 4-cyano phényle, 4-carboxy phényle, 4-hydroxy phényle, 4-amino phényle, 4-hydroxyméthyl phényle ou 4-vinyl phényle et les deux autres groupes parmi $R_a$, $R_b$ ou $R_c$ représentent chacun un atome d'hydrogène.

Selon un troisième aspect particulier, l'invention a pour objet un procédé tel que défini ci-dessus pour lequel dans la formule (I), $R_a$ et $R_b$ représentent chacun un atome d'hydrogène.

Selon un quatrième aspect particulier, l'invention a pour objet un procédé tel que défini ci-dessus pour lequel dans la formule (I), soit $R_a$ et $R_c$, soit $R_b$ et $R_c$, représentent chacun un atome d'hydrogène.

Selon un cinquième aspect particulier de la présente invention, les trois groupes $R_a$, $R_b$ et $R_c$ représentent chacun un atome d'hydrogène.

Selon un sixième aspect particulier, l'invention a pour objet un procédé tel que défini ci-dessus pour lequel dans la formule (I), $R_1$, $R_2$, $R_3$ et $R_4$, identiques ou différents, représentent indépendamment les uns des autres, un radical phényle non substitué ou un radical phényle substitué par un ou plusieurs groupes identiques ou différents choisis indépendamment les uns des autres parmi les radicaux, vinyle, hydroxy, nitro, amino, cyano, carboxy, bromo, chloro, fluoro, iodo, benzyloxy, les radicaux alkyles linéaires ou ramifiés, comportant de 1 à 4 atomes de carbone ou les radicaux alkyloxy linéaires ou ramifiés, comportant de 1 à 4 atomes de carbone, lesdits radicaux alkyles et alkyloxy étant eux-mêmes soit non substitués, soit substitués par un ou plusieurs groupes, bromo, chloro, fluoro ou iodo.

Selon un septième aspect particulier, l'invention a pour objet un procédé tel que défini ci-dessus pour lequel dans la formule (I), $R_5$, $R_6$, $R_7$ et $R_8$, identiques ou différents, représentent indépendamment les uns des autres, un radical alkyle linéaire ou ramifié, comportant de 1 à 4 atomes de carbone et plus particulièrement un radical méthyle ou un radical éthyle.

Selon un huitième aspect particulier, l'invention a pour objet un procédé tel que défini ci-dessus pour lequel dans la formule (I), $R_5$, $R_6$, $R_7$ et $R_8$, sont identiques et représentent chacun un radical méthyle ou un radical éthyle.

Selon un neuvième aspect particulier, l'invention a pour objet, un procédé tel que défini ci-dessus pour lequel dans la formule (I), $R_1$, $R_2$, $R_3$ et $R_4$, sont identiques et représentent chacun un radical phényle.

L'invention a aussi pour objet, un procédé tel que défini aux revendications 9 et 10.

L'invention a aussi pour objet une silice modifiée telle que défie aux revendications 11 et 12, un matériau sol - gel tel que défini aux revendications 13 et 14, et une silice mésoporeuse telle que définie aux revendications 15 et 16.

La partie expérimentale suivante illustre l'invention sans toutefois la limiter.

## 1. Synthèses du ligand

a) - Synthèse à partir d'un dipyrrométhane

La synthèse représentée à la Figure 3, est effectuée par condensation de deux équivalents de carboxypyrrole sur un dipyrrométhane approprié avec catalyse acide. On obtient aisément un intermédiaire de type biladiène "a - c", qui se cyclise sous l'action d'une base avant d'être soumis à une réaction d'oxydation pour former le corrole.[3,4]

b) - Synthèse directe

Dans certaines conditions, il est possible de synthétiser directement en une seule étape le macrocycle corrole. Cette réaction[5], représentée à la Figure 4, est réalisée sans solvant et peut-être assistée par un support solide de type alumine.

c) - Synthèse par condensation d'un dipyrrométhane et d'un dipyrrylsulfure

Cette méthode, représentée à la Figure 5, consiste à opposer à un dipyrrométhane, un dipyrrylsulfure dans le méthanol en milieu acide[6]. Le composé intermédiaire est un "méso" thiamacrocycle, dont la structure dépend de la nature des substituants en position β - pyrrolique. L'extrusion du soufre peut-être obtenue par thermolyse dans le dichlorobenzène et en présence de triphénylphosphine. Cependant cette voie de synthèse ne permet pas actuellement d'obtenir des rendements élevés en corrole.

**2. Métallation par le cobalt**

a) - Métallation du corrole

La métallation du corrole, représentée à la Figure 6, est aisément effectuée par le diacétate de cobalt dans un mélange $CHCl_3/CH_3OH$ à reflux.

b) - Synthèse directe du complexe par effet template

Une autre voie d'accès, représentée à la Figure 7, utilise l'effet template de l'ion cobalt[7-9] pour obtenir directement le complexe de cobalt. Toutefois cette méthodologie suppose l'accès à un complexe dont l'élément central est porteur d'un ligand axial phosphine qui peut partiellement inhiber la coordination ultérieure du monoxyde de carbone.

**3.- Corroles de Co(III): propriétés d'adsorption des gaz**

a) - Choix des complexes

Les trois complexes de cobalt choisis comme exemples pour étudier les propriétés du monoxyde de carbone et de l'oxygène, sont représentés à la Figure 8. L'affinité du Co(III) pour le monoxyde de carbone va dépendre de la densité électronique présente sur le métal acide de Lewis. Plus l'ion métallique est pauvre en électrons (par la présence de substituants électro-attracteurs sur le corrole), plus son affinité vis-à-vis du monoxyde de carbone doit être grande. Par conséquent, on doit donc s'attendre à une plus grande affinité pour le CO du corrole III, par rapport aux corroles I et II (Figure 9). Cette échelle de réactivité pour les corroles I, II et III sera confirmée d'une part par la mesure de la pression de demi-saturation $P_{1/2}$(CO), et d'autre part par celle de la fréquence de vibration infrarouge de la liaison CO.

b) - Adsorption du monoxyde de carbone

Les mesures d'adsorption de CO par les différents complexes ont été réalisées à 21°C, sur un appareil ASAP 2010 Micromeritics™. Les données expérimentales obtenues ont été traitées par un ajustement non linéaire, suivant une loi de type Langmuir :

$$V = (V_m \,.\, K \,.\, P) / (1+KP)$$

formule dans laquelle $V_m$ est le volume maximum de gaz coordonné sur le Co(III), K est la constante de stabilité correspondant à l'équilibre :

$$LCo(III) + CO \rightleftharpoons LCo(CO)$$

et P est la pression partielle de gaz.

Dans tous les cas, deux isothermes de Langmuir sont nécessaires pour rendre compte du phénomène d'adsorption qui doit avoir pour origine, une accessibilité différente des sites cobalt à l'état solide, ainsi qu'il est représenté à la Figure 10. Sur cette figure, on remarque que, même si les sites cobalt sont identiques d'un point de vue chimique, ils ne le sont pas d'un point de vue "géographique", c'est à dire que les sites actifs n'ont pas tous la même accessibilité pour le monoxyde de carbone. En effet par la voie 2, le monoxyde de carbone doit d'abord diffuser dans le solide avant de pouvoir se chimisorber. Pour rendre compte de ce phénomène, il a donc été nécessaire de mettre en jeu deux $P_{1/2}$ (CO), soit deux isothermes de Langmuir.

(i) - Corrole I

Le complexe I ne fixe pas le monoxyde de carbone à l'état solide. Ce résultat est particulièrement important puisqu'il démontre que l'adsorption du monoxyde de carbone n'est pas uniquement attribuable à une espèce Co(III). Cette observation est en accord avec les résultats de la littérature, pour lesquels aucune coordination du monoxyde de carbone n'a été mise en évidence avec un complexe de Co(III). Dans le cas présent le complexe I n'est pas suffisamment acide de Lewis pour permettre la création d'une liaison Co(III)-CO.

(ii) - Corrole II

L'isotherme d'adsorption du monoxyde de carbone par le complexe II est représenté à la Figure 11. Le volume total de monoxyde de carbone à une atmosphère est de 4,11 $cm^3.g^{-1}$, soit un rendement de 10% par rapport au nombre de sites potentiels de la molécule. Ce résultat est en accord avec une faible accessibilité des sites Co(III) à l'intérieur du composé solide. Une dispersion de corrole dans un film de type Langmuir-Blodgett, ou l'immobilisation du complexe sur un support solide de type silice ou polymère organique, par exemple, devrait rendre les sites plus accessibles. Cependant pour une application de type capteur, quelque soit le volume de CO adsorbé, la limite de détection du capteur sera déterminée par la pression partielle de CO à partir de laquelle intervient la chimisorption du gaz sur le corrole. Dès les faibles pressions de gaz, le corrole II fixe le CO. La détermination des constantes thermodynamiques a été effectuée par la méthode d'ajustement des moindres carrés non linéaires avec deux isothermes de Langmuir (Figure 12). Une très bonne adéquation entre les valeurs expérimentales et l'isotherme calculée est obtenue à partir de deux isothermes de Langmuir. Il en résulte un $P_{1/2}$(CO) de 20,6 Torr pour la première isotherme de Langmuir, indiquant une bonne affinité du complexe II pour le CO. Cette isotherme traduit la chimisorption de CO sur des sites de Co(III) facilement accessibles, alors que la deuxième isotherme de Langmuir rend compte à la fois de la diffusion et de l'adsorption du gaz sur les sites difficilement accessibles. C'est pourquoi le $P_{1/2}$(CO) pour la seconde isotherme est dans ce cas très élevé : 1303 Torr. Nous ne tiendrons compte pour la suite que du $P_{1/2}$(CO) relatif à la première isotherme de Langmuir. Il est important de noter que l'adsorption de CO correspond bien à un phénomène de chimisorption au niveau de l'atome de cobalt. Pour cela nous avons étudié les propriétés d'adsorption du CO sur le corrole IV, représenté à la Figure 13, dont les sites potentiels de fixation du gaz sont bloqués par deux molécules d'imidazole. Le corrole IV ne fixe pas le monoxyde de carbone, même à des pressions de 850 Torr en CO. Le spectre infrarouge de ce composé ne présente pas de bandes de vibration caractéristiques du CO fixé sur un métal. Ces deux résultats confirment que le CO est bien lié au Co(III) dans le corrole II. En vue d'une application comme capteur de monoxyde de carbone, il est nécessaire que le processus de fixation du gaz soit réversible. Après un dégazage pendant 12h, sous $10^{-3}$ Torr du composé II/CO, une deuxième isotherme a été enregistrée. Cette isotherme et celle correspondant à la première adsorption sont représentées sur la Figure 14. Cette figure met en évidence la réversibilité totale du système vis-à-vis de CO, puisque le volume de CO adsorbé après le deuxième cycle, est identique à celui mesuré après le premier cycle. Cette réversibilité totale est confirmée par l'analyse infrarouge en mode de réflexion diffuse du composé II/CO (Figure 15). L'analyse infrarouge confirme la bonne réversibilité du système. En effet, sur la Figure 15, on observe bien une désorption progressive du CO lorsque le complexe est remis à l'air (spectres 1 à 5). Cette désorption résulte de la diminution de la pression partielle en CO dans le système. Elle devient ensuite totale lorsque le corrole II est dégazé en 5 mn sous 3 Torr, puisqu'en infrarouge aucune bande n'apparaît à 2045 $cm^{-1}$ (Spectre 6).

(iii) - Corrole III

La Figure 16 représente l'isotherme d'adsorption du monoxyde de carbone pour le corrole III, ainsi que l'ajustement obtenu avec deux isothermes de Langmuir. Comme pour les composés précédents, une très bonne adéquation est observée entre les valeurs expérimentales et l'ajustement obtenu à partir de deux isothermes de Langmuir. La première valeur de $P_{1/2}$(CO) est de 2,85 Torr ce qui indique une excellente affinité du complexe III pour le CO. Si l'on compare ce résultat avec celui obtenu pour le corrole II ($P_{1/2}$(CO) = 20,6 Torr), on constate une affinité accrue du corrole III pour le CO par rapport au corrole II. Ceci confirme l'hypothèse d'une plus grande affinité vis-à-vis du CO pour les complexes dont l'atome Co(III) est appauvri en électrons (voir Figure 9). L'affinité pour le monoxyde de carbone est donc modulable à souhait par ajout de substituants plus ou moins électro-attracteurs à la périphérie du macrocycle corrole. La réversibilité de l'adsorption de CO a également été testée sur quatre cycles d'adsorption - désorption pour le corrole III. Les isothermes correspondantes sont présentées sur la Figure 17. On observe une réversibilité partielle au fur et à mesure des cycles d'adsorption - désorption. Le volume total de CO adsorbé à 850 Torr est de 5,22-5,34 $cm^3.g^{-1}$ pour les deux premiers cycles, et baisse à 3,80-3,98 $cm^3.g^{-1}$ pour le troisième et quatrième cycle. Ce résultat peut s'expliquer par le fait que tous les sites n'ont peut-être pas été libérés entre le deuxième et le troisième cycle étant donné l'affinité importante du corrole III pour le monoxyde de carbone. Il faut toutefois remarquer que la valeur de $P_{1/2}$ (CO) varie peu selon les cycles. Cette réversibilité partielle est confirmée par l'analyse infra - rouge en mode de réflexion diffuse du composé III / CO. Même après un dégazage pendant 5 mn sous 3 Torr, la bande à 2078,8 $cm^{-1}$ (relative à la vibration CO) n'a pas complètement disparu. Ceci met bien en évidence la solidité de la liaison Co(III)-CO dans le cas du corrole III/CO.

**4. Sélectivité $CO/O_2$ et $CO/N_2$**

De nombreuses molécules sont connues pour leurs propriétés de fixation du monoxyde de carbone, mais dans tous les cas elles fixent aussi l'oxygène. Cette absence de sélectivité (nécessaire pour l'utilisation en tant que capteur)

rend impossible leur mise en oeuvre pour un procédé de détection de CO dans une atmosphère ambiante. De plus, les complexes de cobalt ou de fer, connus pour leur capacité à coordonner $O_2$ ou CO, se révèlent être instables à l'air au cours du temps en raison de la présence d'oxygène et d'humidité. Leur application en tant que capteur est donc impossible. Nous présentons sur la Figure 18, les isothermes d'adsorption de CO, $O_2$ et de $N_2$ pour le corrole II. Ils font apparaître les points suivants :

1- la quantité de $N_2$ ou $O_2$ fixée est très faible par rapport au volume de CO adsorbé, surtout dans les faibles pressions.
2- les isothermes d'adsorption de l'azote et de l'oxygène sont identiques ; ceci indique que le phénomène mis en jeu correspond à de la physisorption.

D'un point de vue chimique la sélectivité CO / $O_2$ ou CO / $N_2$ du complexe est donc infinie. Cependant si l'on considère cette sélectivité comme étant le rapport entre les volumes adsorbés des différents gaz à une pression donnée, elle est de l'ordre de 50 pour des pressions inférieures à 5 Torr (insert de la Figure 18). Cette sélectivité CO/$O_2$ ou CO/$N_2$ est nettement plus importante dans le cas du corrole III, puisque la quantité de CO adsorbée est plus importante dès les faibles pressions. La Figure 19 représente les isothermes d'adsorption de CO, $O_2$ et de $N_2$ pour le corrole III, ainsi que les deux courbes de sélectivité. La sélectivité du composé est remarquable dans les faibles pressions, puisqu'elle est de l'ordre de 300 pour des pressions inférieures à 5 Torr, soit six fois celle du corrole II. Ceci résulte simplement de la plus forte affinité du corrole III pour le CO. Le rapport des $P_{1/2}$(CO) pour les deux complexes révèle un facteur 7 en faveur du corrole III. Une telle sélectivité à l'état solide CO/$O_2$ est sans précédent dans la littérature et met en avant l'intérêt de telles molécules dans le domaine des capteurs. Ces résultats sont comparés avec ceux obtenus pour une porphyrine de fer(II) de type "picket fence" connue depuis plus de 20 ans pour coordonner, en solution organique, $O_2$ et CO avec une sélectivité très importante vis-à-vis de CO.[1,10]

**5. Picket fence de Fe(II) : propriétés d'adsorption des gaz**

Nous avons présenté sur la Figure 1, la structure de la "picket fence" porphyrine de fer (II) ((To-PivPP)Fe(1,2-$Me_2$Im)) avec une base de type imidazole coordonnée sur l'atome de fer. La coordination de CO ou $O_2$ s'effectue sur le site vacant du fer(II) dans la cavité porphyrinique[11]. Ce complexe porphyrinique est en effet à ce jour l'un des meilleurs modèles synthétiques de la myoglobine et de l'hémoglobine, puisqu'il coordonne réversiblement l'oxygène[1,10,12,13]. Cette coordination implique un transfert électronique du métal vers l'oxygène générant par là-même un anion superoxyde et l'oxydation formelle du fer(II) en fer(III). Ainsi, les centres métalliques capables de fixer une molécule d'oxygène doivent avoir un site de coordination vacant et un bas degré d'oxydation. La formation de l'espèce oxygénée est stabilisée d'une part par la présence d'une cavité distale (site de coordination opposé à la base) et d'autre part par l'existence de liaisons hydrogène au sein de la cavité entre les groupements amide des piquets pivaloyle et l'anion superoxyde. En modifiant ces deux paramètres, il est possible de faire varier considérablement la sélectivité de ces complexes vis-à-vis de l'oxygène et du monoxyde de carbone. Comme nous l'avons signalé, cette molécule est un des composés possédant la plus grande affinité en solution pour le CO. Elle fixe aussi en solution l'oxygène, mais de manière moins forte. Les valeurs des constantes thermodynamiques relatives à la fixation de CO et $O_2$ par ce complexe sont reportées dans le Tableau 1, ci -dessous.

**Tableau 1** : constante de stabilité de (T*o*-PivPP)Fe(1,2-$Me_2$Im) pour $O_2$ et CO.

| | $P_{1/2}O_2$ (Torr) | $P_{1/2}CO$ (Torr) | M = $P_{1/2}O_2/P_{1/2}CO$ | Conditions |
|---|---|---|---|---|
| (To-PivPP)Fe(1,2-$Me_2$Im) | 38 | $8,9 10^{-3}$ | 4280 | Toluène, 25°C |

Le rapport M, représentatif de la sélectivité CO / $O_2$, est un des plus élevés à ce jour. Cependant ces valeurs ont été obtenues en solution, et n'ont jamais été relevées à l'état solide pour le monoxyde de carbone, et une seule publication relate la coordination de l'oxygène.[14] sur la "picket fence" de fer(II) à l'état solide. Il était donc important de réaliser une étude complète à l'état solide en effectuant des séries de mesures analogues à celles effectuées sur les corroles.

(i) - Adsorption de CO

La Figure 20 représente l'isotherme d'adsorption du monoxyde de carbone à l'état solide pour (To-PivPP)Fe (1,2-$Me_2$Im), ainsi que l'ajustement obtenu avec deux isothermes de Langmuir. Une valeur de $P_{1/2}$(CO) de 0,63 Torr a été calculée pour la première isotherme de Langmuir, indiquant une excellente affinité de (To-PivPP)Fe(1,2-$Me_2$Im) pour le CO. Comme dans le cas des complexes de corroles, la deuxième isotherme de Langmuir rend compte à la fois

de la diffusion et de l'adsorption du gaz sur des sites moins accessibles. Le $P_{1/2}$(CO) est cependant relativement bas : 23 Torr. Ceci traduit une bonne affinité de ces sites "moins accessibles" pour le CO. Le volume total adsorbé à P = 850 Torr est de 9,29 $cm^3.g^{-1}$, ce qui représente 50% des sites actifs. Nous avons également étudié la réversibilité de (To-PivPP)Fe(1,2-$Me_2$Im) vis-à-vis de CO (voir Figure 21). Dans des conditions de dégazage relativement douces (50°C), la fixation de CO sur (To-PivPP)Fe(1,2-$Me_2$Im) n'est pas totalement réversible (cycles 1-3, Figure 21). Ceci s'explique par une liaison très forte existant entre le fer et le CO. En revanche après dégazage à 120°C pendant 12h, le volume adsorbé total est légèrement supérieur à celui obtenu lors du premier cycle (isothermes 1 et 4). Il faut aussi noter que pour les quatre cycles, la valeur de $P_{1/2}$(CO) est comprise entre 0,3 et 0,6 Torr. La grande stabilité de la liaison Fe-CO est confirmée par l'analyse infrarouge en mode de réflexion diffuse de (T*o*-PivPP)Fe(1,2-$Me_2$Im)/CO (Figure 22). Pour des composés à liaison Fe-CO, la bande correspondant à la vibration de la liaison CO apparaît à basse fréquence, ce qui indique que la liaison Fe-CO est forte, en raison d'une rétrodonation $\Pi$ très marquée du métal vers le ligand.

(ii) - Adsorption de $O_2$

La Figure 23 représente l'isotherme d'adsorption de l'oxygène à l'état solide pour (T*o*-PivPP)Fe(1,2-$Me_2$Im) ainsi que l'ajustement obtenu avec deux isothermes de Langmuir. Une valeur $P_{1/2}(O_2)$ de 34,2 Torr a été calculée pour la première isotherme de Langmuir, indiquant une bonne affinité du composé pour $O_2$. Cette affinité est nettement moins importante que pour le CO ($P_{1/2}$(CO) = 0,63 Torr ; Figure 23). On retrouve bien ici un comportement en accord avec celui observé en solution. Le volume total adsorbé à P = 850 Torr est de 9,29 $cm^3.g^{-1}$ (résultat identique à celui obtenu pour le CO), ce qui représente 50% des sites actifs. De même nous avons étudié la réversibilité de (T*o*-PivPP)Fe(1,2-$Me_2$Im) vis-à-vis de $O_2$ (voir Figure 24). A l'inverse de ce qui a été observé pour le monoxyde de carbone, la réversibilité semble meilleure pour l'oxygène, puisque le volume total adsorbé diminue peu entre chaque cycle. Ceci s'explique par une liaison moins forte entre $O_2$ et le fer, des conditions douces de dégazage sont alors suffisantes pour désorber $O_2$. Une réversibilité totale devrait être obtenue par chauffage sous vide.

**6. Comparaison entre les corroles (Co(III)) et la picket fence (Fe(II))**

Nous présentons dans le Tableau 2 les valeurs obtenues pour les corroles de cobalt (III) et pour (T*o*-PivPP) Fe(1,2-$Me_2$Im).

**Tableau 2 :** comparaison entre les corroles de cobalt et (T*o*-PivPP)Fe(1,2-$Me_2$Im).

| Composés | $P_{1/2}O_2$ (Torr) | $P_{1/2}$CO (Torr) | M = ($P_{1/2}O_2$/$P_{1/2}$CO) | Réversibilité |
|---|---|---|---|---|
| (To-PivPP)Fe(1,2-$Me_2$Im). | 34,17 | 0,63 | 54 | A |
| Corrole II | 391,35 | 20,6 | 19 | B |
| Corrole III | 263,78 | 2,85 | 93 | C |
| (A : partielle à 20°C, totale à 120°C; B: totale à 20°C; C : partielle à 20°C) | | | | |

Il est important de rappeler que l'oxygène adsorbé par les corroles correspond à un phénomène de physisorption. A l'inverse, pour (To-PivPP)Fe(1,2-$Me_2$Im), il intervient une liaison chimique entre le fer et $O_2$. La sélectivité "chimique" est donc infinie pour les corroles II et III. Cependant même en tenant compte de $O_2$ physisorbé, le corrole III présente une sélectivité nettement supérieure à celle obtenue pour la porphyrine. De plus il est manipulable à l'air et ne se dégrade pas en présence d'humidité. Ces résultats sans précédent, mettent en évidence les potentialités d'une telle molécule dans différents domaines d'application, et en particulier dans celui des capteurs de monoxyde de carbone.

**7. Partie experimentale**

(i) - Synthèse du corrole I

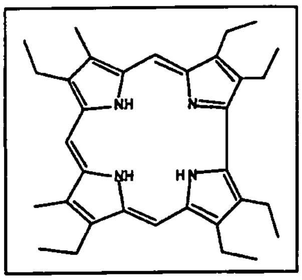

A une suspension de 2,38 g (7,5 mmoles) de 5,5'-dicarboxy-3,3'-diéthyl-4,4'-diméthyl dipyrrylméthane et de 2,26g (15 mmoles) de 3,4-diéthyl-2-formylpyrrole [ou 2,15 g (7,5 mmoles) de 3,3'-diéthyl-5,5'-diformyl-4,4'-diméthyl dipyrrylméthane et 2,51 g (15 mmoles) de 2-carboxy-3,4-diéthylpyrrole] dans 120 ml d'éthanol à reflux, on ajoute 15 ml d'acide bromhydrique à 33 % dans l'acide acétique. Le mélange réactionnel est alors porté à reflux pendant 10 minutes. Après refroidissement de la solution, le biladiène-a,c (intermédiaire réactionnel) précipite lors de l'addition de 150 ml d'ether diéthylique. Le biladiène-a,c est filtré, rincé à l'éther et séché. On obtient une poudre violette avec un rendement de 70 %. Le biladiène-a,c est ensuite dissous dans 500 ml de méthanol saturé en $NaHCO_3$ et la solution est agitée 10 minutes. 730 mg de p-chloranil sont ensuite additionnés et, après 10 minutes d'agitation supplémentaire, on ajoute 7 ml d'hydrate d'hydrazine à 50 % dans l'eau. Après 10 minutes d'agitation, le corrole précipite sous forme d'une poudre rose - violette. Le corrole est alors filtré et lavé abondamment à l'eau. Le corrole est ensuite recristallisé dans un mélange $CH_2Cl_2/CH_3OH$, filtré et séché.

Le corrole I est obtenu avec un rendement de 65 %.

RMN du proton ($CDCl_3$) (δ en ppm) : -2,93 (s, 3H, N**H**); 1,84 (t, 6H, C**H**$_3$); 1,87 (t, 6H, C**H**$_3$); 1,89 (t, 6H, C**H**$_3$); 3,38 (s, 6H, C**H**$_3$); 3,90 (q, 4H, $CH_2$); 3,95 (q, 4H, $CH_2$); 4,06 (q, 4H, $CH_2$); 9,51 (s, 1 H, **H-10**); 9,53 (s, 2H, **H-5,15**).

Spectrométrie infrarouge (KBr ; ν en $cm^{-1}$) : 3350 (NH); 2961 (CH); 2928 (CH); 2867 (CH).

Spectrométrie de masse (El): m/z = 494 ($M^{+\bullet}$) (100)

Analyse centésimale pour $C_{33}H_{42}N_4$ : calculée : C 80,1%; H 8,6%; N 11,3% trouvée : C 79,7%; H 8,6%; N 11,4%

Spectrophotométrie UV-Visible ($CH_2Cl_2$) : $\lambda_{max}$, nm ($\varepsilon.10^{-3}$ $M^{-1}.cm^{-1}$) : 395 (126,5); 407 (101,2); 549 (18,9); 593 (23,6).

(ii) - Synthèse du corroie II

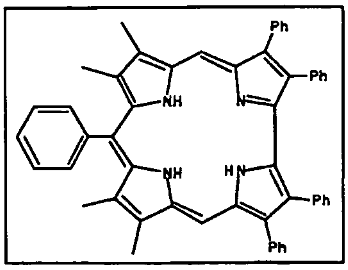

Après dissolution de 4,72 g (12,8 mmoles) de 5,5'-dicarboxy-3,3',4,4'-tetraméthyldipyrrltoluène dans 200 ml d'acide trifluoroacétique, la solution rouge est agitée à température ambiante pendant 5 minutes. Une solution de 7g (25,6 mmoles) de 3,4-diphényl-2-formylpyrrole dans 200 ml de méthanol est alors additionnée goutte-à-goutte. L'agitation est maintenue durant 15 minutes et 70 ml d'acide bromhydrique à 33 % dans l'acide acétique sont ajoutés. Le mélange réactionnel est agité 15 minutes puis les solvants sont évaporés. On obtient un solide ayant des reflets verts (biladiène-a,c). Le biladiène-a,c est alors dissous dans 11 de méthanol saturé en $NaHCO_3$ et agité pendant 15 minutes. 4,3 g de p-chloranil sont alors ajoutés et le milieu réactionnel est de nouveau agité 15 minutes. Enfin, 43 ml d'hydrate d'hydrazine à 50 % dans l'eau sont additionnés et, après 15 minutes d'agitation supplémentaire, les solvants sont évaporés. Le solide obtenu est repris dans le dichlorométhane et lavé à l'eau jusqu'à pH neutre. La phase organique est ensuite séchée sur $MgSO_4$, filtrée et évaporée. Le solide obtenu est passé sur colonne chromatographique d'alumine (éluant : 100 % $CH_2Cl_2$). La fraction violette qui élue en front de solvant est collectée et évaporée. Le corrole est ensuite recristallisé dans un mélange $CH_2Cl_2/CH_3OH$, filtré et séché.

Le corrole II est obtenu avec un rendement de 10 %.

RMN du proton ($CDCl_3$) (δ en ppm) : 2,22 (s, 6H, C**H**$_3$); 3,17 (s, 6H, C**H**$_3$); 6,57-7,95 (m, 25H, Ar-H); 9,40 (s, 2H, H-**5,15**).
Spectrométrie de masse (LSIMS) : m/z = 734 ($M^{+\bullet}$) (100).
Spectrophotométrie UV-Visible ($CH_2Cl_2$) : $\lambda_{max}$, nm ($\varepsilon.10^{-3}$ $M^{-1}.cm^{-1}$) : 418 (85,3); 566 (14,3); 604 (12,1).

(iii) - Synthèse du corrole III

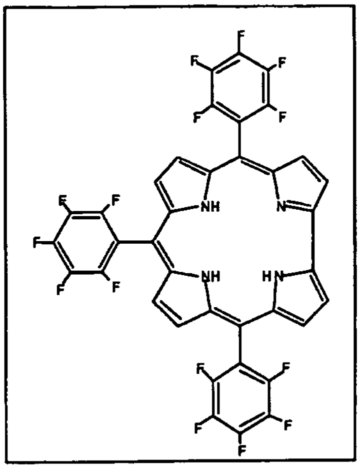

La synthèse décrite ici a été développée par l'équipe du Professeur Gross en 1999[5]. Cette synthèse permet d'obtenir, en une seule étape, un corrole à partir de deux produits commerciaux à moindre coût. La synthèse est assisté par un support solide de type alumine et est réalisée sans solvant. A 200 mg d'alumine préalablement broyée et déshydratée sont ajoutés 2,94 g (15 mmoles) de pentafluorobenzaldéhyde et 0,967 ml (15 mmoles) de pyrrole. Le mélange réactionnel est agité à 60°C pendant 4 heures. Le solide obtenu est alors dissous dans le dichlorométhane et 200 mg de DDQ sont additionnés. La solution violette est évaporée et le solide obtenu est passé sur colonne chromatographique d'alumine (éluant : 100 % $CH_2Cl_2$). La fraction violette qui migre en front de solvant est collectée puis évaporée. Le corrole est ensuite recristallisé dans un mélange $CH_2Cl_2/CH_3OH$, filtré et séché.

Le corrole III est obtenu avec un rendement de 15 %.
RMN du proton ($CDCl_3$) (δ en ppm) : 7-9 (4dd, 8H, Hpyr.).
Spectrométrie de masse (LSIMS) : m/z = 796 ($M^{+\bullet}$) (100).

(iv) - Métallation des corroles I, II et III

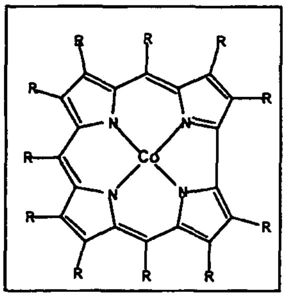

Le corrole est dissous dans le chloroforme et est porté à reflux. On ajout à cette solution, 1,2 équivalents de $Co(O_2CCH_3)_2$ dissous dans le minimum de méthanol. Le milieu réactionnel est agité pendant 10 minutes à reflux puis les solvants sont évaporés. Le solide est ensuite repris dans le dichlorométhane et lavé à l'eau. La phase organique est ensuite séchée sur $MgSO_4$, filtrée puis évaporée. Le corrole de cobalt obtenu est recristallisé dans un mélange $CH_2Cl_2/CH_3OH$, filtré et séché.

Les corroles de cobalt sont obtenus avec des rendements supérieurs à 90 %. Les principales caractéristiques analytiques des complexes obtenus sont résumées dans le tableau suivant :

| (Corrole)Co | UV-Visible $\lambda_{max}$ ($\varepsilon$. $10^{-4}$ $M^{-1}$ $cm^{-1}$) | MALDI-TOF |
|---|---|---|
| (Corrole I)Co | 381 (7,7); 502 (0,9) | m/z = 550 |
| (Corrole II)Co | 399 (4,7); 531 (1,2) | m/z = 788 |
| (Corrole III)Co | 394 (2,8); 501 (1,0) | m/z = 852 |

(v) - Accès à des matériaux hybrides organiques - inorganiques

a) Greffage sur silice

Le greffage sur silice est réalisé par ancrage du macrocycle corrole, métallé ou non, par l'intermédiaire d'un bras fonctionnalisé, sur les fonctions silanol libres du gel de silice selon le processus réactionnel suivant :

- La première étape consiste en l'addition du bras fonctionnalisé sur un seul des groupes aryle $R_a$, $R_b$ ou $R_c$ du corroie (Schéma 1). Ce bras peut être fonctionnalisé par les groupements vinyle, amino ou par un atome d'halogène. La longueur de ce bras peut varier de zéro atome de carbone (le groupe fonctionnel est alors directement fixé sur le motif trialkoxysilane) à quatre atomes maximum. Le reste aryle sur lequel vient se fixer le bras espaceur est fonctionnalisé en position $R_9$, $R_{10}$ $R_{12}$ ou $R_{13}$ par le groupement adéquat selon le Schéma 1.
- La seconde étape correspond au greffage du macrocycle fonctionnalisé sur le gel de silice par réaction du motif trialkoxysilyle sur une, deux ou trois fonctions silanol accessibles de la silice (Schéma 2).

b) Formation directe du matériau par la technique Sol - Gel

Les matériaux sol - gels sont synthétisés en procédant de la manière suivante :

Voie 1 : Polycondensation de corroles ou métallocorroles mono, di et tri- substitués en position meso ($R_a$, $R_b$ et $R_c$) par des espaceurs à terminaison trialkoxysilyle (Schéma 3).
La fonctionnalisation préalable des groupes aryle $R_a$, $R_b$ et $R_c$ procèdera selon le même protocole que celui décrit précédemment pour le greffage sur le gel de silice (Schéma 1). La polycondensation sera réalisée par réaction du corrole ou métallocorrole trisubstitué préalablement mentionné avec la quantité stoechiométrique d'eau, en présence d'un catalyseur (fluorure, acide ou base) dans tout solvant organique permettant la solubilisation du milieu réactionnel.
Voie 2: Co-polycondensation de corroles ou métallocorroles mono di et tri- substitués en position meso ($R_a$, $R_b$ et $R_c$) par des bras à terminaisons trialkoxysilyle avec un tétraalkoxysilane (formation d'un cogel) (Schéma 3).

La fonctionnalisation du corrole ou métallocorrole est rigoureusement identique à celle décrite précédemment. La co-polycondensation sera réalisée par réaction du corrole ou métallocorrole trisubstitué préalablement mentionné avec le tétraalkoxysilane et la quantité stoechiométrique d'eau, en présence d'un catalyseur (fluorure, acide ou base) dans tout solvant organique permettant la solubilisation du milieu réactionnel.

c) Greffage direct sur un matériau de type MTS

Cette famille de matériaux, découverte par Beck en 1992, est nommée MTS (Mesoporous Templated Silica) et deux représentants de cette famille, nommés HMS (Hexagonal Mesoporous Silica) ou MSU, correspondent à nos besoins. L'organisation du matériau HMS ou MSU s'opère par polycondensation d'un précurseur hydrolysable en présence d'un agent surfactant neutre tel qu'une amine primaire (HMS) de $C_8$ à $C_{18}$ ou un oxyde de polyéthylène (MSU). L'agent structurant est facilement éliminé par extraction à l'éthanol. L'élimination du surfactant libère ainsi des canaux hexagonaux dont le diamètre moyen est régulier et présente une accessibilité remarquable à diverses molécules. L'ancrage du motif corroie à l'intérieur des pores du matériau peut être effectué par réaction du macrocycle mono ou difonctionnalisé par un enchaînement comportant un groupe trialkoxysilane terminal avec les groupes silanol libres du matériau (Schéma 4). La fonctionnalisation préalable des groupes aryle $R_a$ et/ou $R_b$ et/ou $R_c$ est réalisée selon le même protocole que celui mentionné précédemment (Schéma 1).

Références

(1) J. P. Collman, L. FU, *Acc. Chem. Res.,* **1999,** *32*, 455-463.
(2) R. Paolesse, *Heteroporphyrins, Expanded Porphyrins and Related Macrocycles,* in *The Porphyrin Handbook, Vol. 2*, Smith, K. M.;. Kadish, K. M.; Guilard, R., Academic Press, New-York 2000, p. 201-233.
(3) A. W. Johnson, I. T. Kay, *Proc. Chem. Soc.,* **1964**,, 89-90.
(4) A. W. Johnson, I. T. Kay, *Proc. Roy. Soc. A,* **1965,** *288*, 334-341.
(5) Z. Gross, N. Galili, L. Simkhovich, I. Saltsman, M. Botoshansky, D. Bläser, R. Boese, I. Goldberg, *Org. Lett.*, **1999,** *1*, 599-602.
(6) M. J. Broadhurst, R. Grigg, A. W. Johnson, *J. Chem. Soc., Perkin I*, **1972, ,** 1124-1135.
(7) R. Paolesse, S. Licoccia, M. Fanciullo, E. Morgante, T. Boschi, *Inorg. Chim. Acta,* **1993**, *203*, 107-114.
(8) R. Paolesse, S. Licoccia, G. Bandoli, A. Dolmella, T. Boschi, *Inorg Chem,* **1994,** *33*, 1171-1176.
(9) S. Licoccia, E. Tassoni, R. Paolesse, T. Boschi, *Inorg Chim Acta,* **1995**, *235*, 15-20.
(10) J. P. Collman, J. I. Brauman, B. L. Iverson, J. L. Sessler, R. M. Morris, Q. H. Gibson, *J. Am. Chem. Soc.,* **1983**, *105*, 3052-3064.
(11) J. P. Collman, R. R. Gagne, C. A. Reed, T. R. Halbert, G. Lang, W. T. Robinson, *J. Am. Chem. Soc.,* **1975**, *97*, 1427-1439.
(12) J. P. Collman, J. I. Brauman, T. R. Halbert, K. S. Suslick, *Proc. Natl. Acad. Sci. USA*, **1976**, *73*, 3333-3337.
(13) J. P. Collman, *Inorg. Chem.,* **1997**, *36*, 5145-5155.
(14) J. P. Collman, J. I. Brauman, K. S. Suslick, *J. Am. Chem. Soc.,* **1975**, *97*, 7185-7186.

## Revendications

**1.** Procédé de séparation du monoxyde de carbone, d'un mélange de gaz en contenant **caractérisé en ce que** ledit mélange est mis en contact avec,
soit un composé de formule (II) :

$$G(Y)_{\alpha},$$

dans laquelle G représente un cation organométallique de formule (II') :

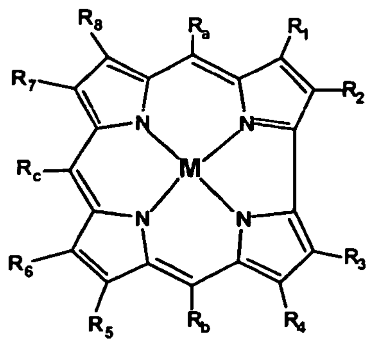

obtenu formellement à partir d'un composé de formule (I) :

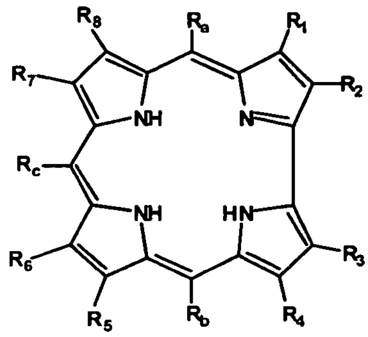

dans laquelle :

$R_1$, $R_2$, $R_3$ et $R_4$, identiques ou différents, représentent indépendamment les uns des autres, un atome d'hydrogène, un radical alkyle linéaire ou ramifié, comportant de 1 à 4 atomes de carbone, un radical phényle non substitué ou un radical phényle substitué par un ou plusieurs groupes, identiques ou différents, choisis indépendamment les uns des autres parmi les radicaux, vinyle, hydroxy, nitro, amino, cyano, carboxy, bromo, chloro, fluoro, iodo, benzyloxy, les radicaux alkyles linéaires ou ramifiés, comportant de 1 à 4 atomes de carbone ou les radicaux alkyloxy linéaires ou ramifiés, comportant de 1 à 4 atomes de carbone, lesdits radicaux alkyles et alkyloxy étant eux mêmes soit non substitués, soit substitués par un ou plusieurs groupes, bromo, chloro, fluoro ou iodo ;
$R_5$, $R_6$, $R_7$ et $R_8$, identiques ou différents, représentent indépendamment les uns des autres, un atome d'hydrogène ou un radical alkyle linéaire ou ramifié, comportant de 1 à 4 atomes de carbone ; et
soit $R_a$, $R_b$ et $R_c$, identiques ou différents, représentent indépendamment les uns des autres, un atome d'hydrogène, un radical phényle non substitué ou un radical phényle substitué par un ou plusieurs groupes identiques ou différents choisis indépendamment les uns des autres parmi les radicaux, vinyle, hydroxy, nitro, cyano, carboxy, amino, bromo, chloro, fluoro, iodo, benzyloxy ou hydroxyméthyle, les radicaux alkyles linéaires ou ramifiés, comportant de 1 à 4 atomes de carbone ou les radicaux alkyloxy linéaires ou ramifiés, comportant de 1 à 4 atomes de carbone, lesdits radicaux alkyles et alkyloxy étant eux-mêmes soit non substitués, soit substitués par un ou plusieurs groupes, bromo, chloro, fluoro ou iodo,
soit les trois groupes $R_a$, $R_b$ et $R_c$ représentent chacun un atome d'hydrogène, étant entendu que dans ce cas au moins un des radicaux $R_1$, $R_2$, $R_3$, $R_4$, $R_5$, $R_6$, $R_7$ ou $R_8$ ne représente pas un atome d'hydrogène,
en substituant M aux hydrogènes des groupes pyrrole dans la formule (I) où M représente un cation métallique choisi parmi les ions du cobalt (Co), du rhodium (Rh), de l'iridium (Ir), du manganèse (Mn), du fer (Fe), du ruthénium (Ru) et de l'osmium (Os) ; Y représente un anion organique ou inorganique et $\alpha$ représente un nombre entier ou décimal de façon à ce que le composé de formule (II), soit électriquement neutre ;
soit avec une silice modifiée par ledit composé de formule (II) dans laquelle au moins un des groupes $R_a$, $R_b$ ou $R_c$ représente un radical phényle non substitué ou un radical phényle substitué, obtenue par introduction de bras espaceurs permettant l'ancrage des cations de formules (II') au gel de silice, qui sont des radicaux divalents, d'une part liés auxdits composés par l'une des positions 2, 3 ou 4 d'un ou de plusieurs des radicaux phényle représentés par au moins un des groupes $R_a$, $R_b$ ou $R_c$, et d'autre part liés à une ou plusieurs des fonctions silanol libres d'un gel de silice par une liaison covalente Si - O ;
soit avec un matériau sol - gel de composé de formule (II), dans laquelle les trois groupes $R_a$, $R_b$ et $R_c$ représentent un radical phényle non substitué ou un radical phényle substitué, obtenu en substituant la position 2, 3 ou 4 desdits radicaux phényle par un radical monovalent possédant un groupe terminal :

$$-Si(OR_{14})_3$$

dans lequel $R_{14}$ représente un radical méthyle, un radical éthyle ou un radical isopropyle.
soit avec une silice mésoporeuse modifiée par un composé de formule (II), dans laquelle au moins un des groupes $R_a$, $R_b$ ou $R_c$ représente un radical phényle non substitué ou un radical phényle substitué, obtenue par introduction de bras espaceurs permettant l'ancrage des cations de formules (II') à la silice mésoporeuse, qui sont des radicaux divalents, d'une part liés auxdits composés de formule (II) par l'une des positions 2, 3 ou 4 d'un seul ou de plusieurs des radicaux phényle représentés par au moins un des groupes $R_a$, $R_b$ ou $R_c$, et d'autre part liés à une ou plusieurs des fonctions silanol libres de la silice mésoporeuse par une liaison covalente

Si - O.

2. Procédé tel que défini à la revendication 1, pour lequel dans la formule (I), un seul des groupes $R_a$, $R_b$ ou $R_c$ représente un radical choisi les radicaux 4-bromo phényle, 4-chloro phényle, 4-iodo phényle, 4-nitro phényle, 4-cyano phényle, 4-carboxy phényle, 4-hydroxy phényle, 4-amino phényle, 4-hydroxyméthyl phényle ou 4-vinyl phényle et les deux autres groupes parmi $R_a$, $R_b$ ou $R_c$ représentent chacun un atome d'hydrogène.

3. Procédé tel que défini à l'une des revendications 1 ou 2, pour lequel dans la formule (I), $R_a$ et $R_b$ représentent chacun un atome d'hydrogène.

4. Procédé tel que défini à l'une des revendications 1 à 3, pour lequel dans la formule (I), soit $R_a$ et $R_c$, soit $R_b$ et $R_c$, représentent chacun un atome d'hydrogène.

5. Procédé tel que défini à l'une des revendications 1 à 4, pour lequel dans la formule (I), $R_1$, $R_2$, $R_3$ et $R_4$, identiques ou différents, représentent indépendamment les uns des autres, un radical phényle non substitué ou un radical phényle substitué par un ou plusieurs groupes identiques ou différents choisis indépendamment les uns des autres parmi les radicaux, vinyle, hydroxy, nitro, amino, cyano, carboxy, bromo, chloro, fluoro, iodo, benzyloxy, les radicaux alkyles linéaires ou ramifiés, comportant de 1 à 4 atomes de carbone ou les radicaux alkyloxy linéaires ou ramifiés, comportant de 1 à 4 atomes de carbone, lesdits radicaux alkyles et alkyloxy étant eux mêmes soit non substitués, soit substitués par un ou plusieurs groupes, bromo, chloro, fluoro ou iodo.

6. Procédé tel que défini à l'une des revendications 1 à 5, pour lequel dans la formule (I), $R_5$, $R_6$, $R_7$ et $R_8$, identiques ou différents, représentent indépendamment les uns des autres, un radical alkyle linéaire ou ramifié, comportant de 1 à 4 atomes de carbone et plus particulièrement un radical méthyle ou un radical éthyle.

7. Procédé tel que défini à l'une des revendications 1 à 6, pour lequel dans la formule (I), $R_5$, $R_6$, $R_7$ et $R_8$, sont identiques et représentent chacun un radical méthyle ou un radical éthyle.

8. Procédé tel que défini à l'une des revendications 1 à 7, pour lequel dans la formule (I), $R_1$, $R_2$, $R_3$ et $R_4$, sont identiques et représentent chacun un radical phényle.

9. Procédé tel que défini à l'une des revendications 1 à 8, **caractérisé en ce qu'**il est mis en oeuvre pour détecter la présence de monoxyde de carbone dans une atmosphère donnée et plus particulièrement dans l'air humide.

10. Procédé tel que défini à l'une des revendications 1 à 9, **caractérisé en ce qu'**il est mis en oeuvre pour séparer le monoxyde de carbone de l'hydrogène dans les procédés de chimie industrielle conduisant à ce mélange.

11. Silice modifiée par un composé de formule (II) apte à la mise en oeuvre du procédé tel que défini à l'une des revendications 1 à 10, dans laquelle au moins un des groupes Ra, Rb ou Rc représente un radical phényle non substitué ou un radical phényle substitué, **caractérisée en ce qu'**elle est obtenue par introduction de bras espaceurs permettant l'ancrage des cations de formules (II') au gel de silice, qui sont des radicaux divalents, d'une part liés aux dits composés par l'une des positions 2, 3 ou 4 d'un ou de plusieurs des radicaux phényle représentés par au moins un des groupes $R_a$, $R_b$ ou $R_c$, et d'autre part liés à une ou plusieurs des fonctions silanol libres d'un gel de silice par une liaison covalente Si - O.

12. Silice modifiée telle que définie à la revendication 11, pour laquelle le radical divalent reliant l'atome de silicium participant à une ou à plusieurs liaisons covalentes Si-O, et la position 2, 3 ou 4 dudit radical phényle, est choisi parmi les radicaux divalents suivants :

$$-CH=CH-, -NH-(CH_2)_n-, -O-(CH_2)_m- \text{ ou } -NH(C=O)-CH_2-NH-(CH_2)_p-,$$

dans lesquels n, m et p, identiques ou différents, représentent indépendamment les uns des autres, un nombre entier supérieur ou égal à 0 et inférieur ou égal à 4.

13. Matériau sol-gel dérivé d'un composé de formule (II) apte à la mise en oeuvre du procédé tel que défini à l'une des revendications 1 à 10, dans laquelle trois groupes $R_a$, $R_b$ et $R_c$ représentent un radical phényle non substitué ou un radical phényle substitué, **caractérisé en ce qu'**il est obtenu en substituant la position 2, 3 ou 4 desdits radicaux phényle par un radical monovalent possédant un groupe terminal :

$-Si(OR_{14})_3$

dans lequel $R_{14}$ représente un radical méthyle, un radical éthyle ou un radical isopropyle..

**14.** Matériau tel que défini à la revendication 13, dans lequel le radical monovalent possédant un groupe terminal -Si $(OR_{14})_3$, est choisi parmi les radicaux suivants :

$-CH=CH-Si(OR_{14})_3$, $-NH-(CH_2)_n-Si(OR_{14})_3$, $-O-(CH_2)_m-Si(OR_{14})_3$ ou

$-NH(C=O)-CH_2-NH-(CH_2)_p-Si(OR_{14})_3$,

dans lesquels n, m et p, identiques ou différents, représentent indépendamment les uns des autres, un nombre entier supérieur ou égal à 0 et inférieur ou égal à 4 et $R_{14}$ représente un radical méthyle, un radical éthyle ou un radical isopropyle.

**15.** Silice mésoporeuse modifiée par un composé de formule (II) apte à la mise en oeuvre du procédé tel que défini à l'une des revendications 1 à 10, dans lesquelles au moins un des groupes $R_a$, $R_b$ ou $R_c$ représente un radical phényle non substitué ou un radical phényle substitué, **caractérisée en ce qu'**elle est obtenue par introduction de bras espaceurs permettant l'ancrage des molécules de formules (I) ou des cations de formules (II') à la silice mésoporeuse, qui sont des radicaux divalents, d'une part liés auxdits composés par l'une des positions 2, 3 ou 4 d'un seul ou de plusieurs des radicaux phényle représentés par au moins un des groupes $R_a$, $R_b$ ou $R_c$, et d'autre part liés à une ou plusieurs des fonctions silanol libres de la silice mésoporeuse par une liaison covalente Si - O.

**16.** Silice mésoporeuse modifiée telle que définie à la revendication 15 dans laquelle le radical divalent reliant l'atome de silicium participant à une ou à plusieurs liaisons covalentes Si-O, et la position 2, 3 ou 4 dudit radical phényle, est choisi parmi les radicaux divalents suivants :

$-CH=CH-$, $-NH-(CH_2)_n-$, $-O-(CH_2)_m-$ ou $-NH(C=O)-CH_2-NH-(CH_2)_p-$,

dans lesquels n, m et p, identiques ou différents, représentent indépendamment les uns des autres, un nombre entier supérieur ou égal à 0 et inférieur ou égal à 4.

## Claims

**1.** Process for the separation of carbon monoxide from a gas mixture comprising it, **characterized in that** the said mixture is brought into contact,
either with a compound of formula (II):

$G(Y)_\alpha$

in which G represents an organometallic cation of formula (II'):

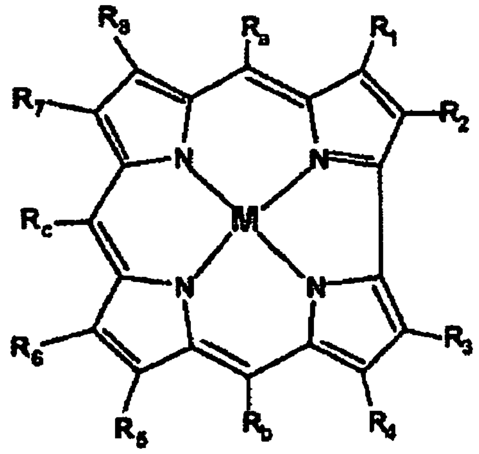

obtained formally from a compound of formula (I):

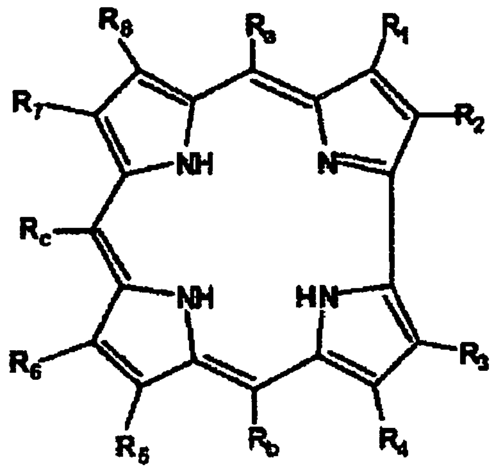

in which:

$R_1$, $R_2$, $R_3$ and $R_4$, which are identical or different, represent, independently of one another, a hydrogen atom, a linear or branched alkyl radical comprising from 1 to 4 carbon atoms, an unsubstituted phenyl radical or a phenyl radical substituted by one or more identical or different groups chosen, independently of one another, from vinyl, hydroxyl, nitro, amino, cyano, carboxyl, bromo, chloro, fluoro, iodo or benzyloxy radicals, linear or branched alkyl radicals comprising from 1 to 4 carbon atoms or linear or branched alkyloxy radicals comprising from 1 to 4 carbon atoms, the said alkyl and alkyloxy radicals themselves either being unsubstituted or substituted by one or more bromo, chloro, fluoro or iodo groups;
$R_5$, $R_6$, $R_7$ and $R_8$, which are identical or different, represent, independently of one another, a hydrogen atom or a linear or branched alkyl radical comprising from 1 to 4 carbon atoms;
either $R_a$, $R_b$ and $R_c$, which are identical or different, represent, independently of one another, a hydrogen atom, an unsubstituted phenyl radical or a phenyl radical substituted by one or more identical or different groups chosen, independently of one another, from vinyl, hydroxyl, nitro, cyano, carboxyl, amino, bromo, chloro, fluoro, iodo, benzyloxy or hydroxymethyl radicals, linear or branched alkyl radicals comprising from 1 to 4 carbon atoms or linear or branched alkyloxy radicals comprising from 1 to 4 carbon atoms, the said alkyl and alkyloxy radicals themselves either being unsubstituted or substituted by one or more bromo, chloro, fluoro or iodo groups,
or the three $R_a$, $R_b$ and $R_c$ groups each represent a hydrogen atom, it being understood that, in this case, at least one of the $R_1$, $R_2$, $R_3$, $R_4$, $R_5$, $R_6$, $R_7$ or $R_8$ radicals does not represent a hydrogen atom,
M being substituted for the hydrogens of the pyrrole groups in the formula (I), where M represents a metal cation

chosen from cobalt (Co), rhodium (Rh), iridium (Ir), manganese (Mn), iron (Fe), ruthenium (Ru) and osmium (Os) ions; Y represents an organic or inorganic anion and $\alpha$ represents an integer or decimal number so that the compound of formula (II) is electrically neutral;

or with a silica modified by the said compound of formula (II) in which at least one of the $R_a$, $R_b$ or $R_c$ groups represents an unsubstituted phenyl radical or a substituted phenyl radical, which silica is obtained by introduction of spacer arms, which make possible the anchoring of the cations of formula (II') to the silica gel, which are divalent radicals bonded, on the one hand, to the said compounds via one of the 2, 3 or 4 positions of one or more of the phenyl radicals represented by at least one of the $R_a$, $R_b$ or $R_c$ groups and bonded, on the other hand, to one or more of the free silanol functional groups of a silica gel via an Si-O covalent bond;

or with a sol-gel material of compound of formula (II) in which the three $R_a$, $R_b$ and $R_c$ groups represent an unsubstituted phenyl radical or a substituted phenyl radical, which material is obtained by substituting the 2-, 3- or 4-position of the said phenyl radicals by a monovalent radical having an end group:

$$-Si(OR_{14})_3$$

in which $R_{14}$ represents a methyl radical, an ethyl radical or an isopropyl radical;

or with a mesoporous silica modified by a compound of formula (II) in which at least one of the $R_a$, $R_b$ or $R_c$ groups represents an unsubstituted phenyl radical or a substituted phenyl radical, which silica is obtained by introduction of spacer arms, which make possible the anchoring of the cations of formula (II') to the mesoporous silica, which are divalent radicals bonded, on the one hand, to the said compounds of formula (II) via one of the 2-, 3- or 4-positions of just one or of several of the phenyl radicals represented by at least one of the $R_a$, $R_b$ or $R_c$ groups and bonded, on the other hand, to one or more of the free silanol functional groups of the mesoporous silica via an Si-O covalent bond.

**2.** Process as defined in Claim 1, for which, in the formula (I), just one of the $R_a$, $R_b$ or $R_c$ groups represents a radical chosen from 4-bromophenyl, 4-chlorophenyl, 4-iodophenyl, 4-nitrophenyl, 4-cyanophenyl, 4-carboxyphenyl, 4-hydroxyphenyl, 4-aminophenyl, 4-(hydroxymethyl)phenyl or 4-vinylphenyl radicals and the other two groups from $R_a$, $R_b$ or $R_c$ each represent a hydrogen atom.

**3.** Process as defined in either of Claims 1 and 2, for which, in the formula (I), $R_a$ and $R_b$ each represent a hydrogen atom.

**4.** Process as defined in one of Claims 1 to 3, for which, in the formula (1), either $R_a$ and $R_c$ or $R_b$ and $R_c$ each represent a hydrogen atom.

**5.** Process as defined in one of Claims 1 to 4, for which, in the formula (I), $R_1$, $R_2$, $R_3$ and $R_4$, which are identical or different, represent, independently of one another, an unsubstituted phenyl radical or a phenyl radical substituted by one or more identical or different groups chosen, independently of one another, from vinyl, hydroxyl, nitro, amino, cyano, carboxyl, bromo, chloro, fluoro, iodo or benzyloxy radicals, linear or branched alkyl radicals comprising from 1 to 4 carbon atoms or linear or branched alkyloxy radicals comprising from 1 to 4 carbon atoms, the said alkyl and alkyloxy radicals themselves either being unsubstituted or substituted by one or more bromo, chloro, fluoro or iodo groups.

**6.** Process as defined in one of Claims 1 to 5, for which, in the formula (I), $R_5$, $R_6$, $R_7$ and $R_8$, which are identical or different, represent, independently of one another, a linear or branched alkyl radical comprising from 1 to 4 carbon atoms and more particularly a methyl radical or an ethyl radical.

**7.** Process as defined in one of Claims 1 to 6, for which, in the formula (I), $R_5$, $R_6$, $R_7$ and $R_8$ are identical and each represent a methyl radical or an ethyl radical.

**8.** Process as defined in one of Claims 1 to 7, for which, in the formula (I), $R_1$, $R_2$, $R_3$ and $R_4$ are identical and each represent a phenyl radical.

**9.** Process as defined in one of Claims 1 to 8, **characterized in that** it is carried out in order to detect the presence of carbon monoxide in a given atmosphere and more particularly in humid air.

**10.** Process as defined in one of Claims 1 to 9, **characterized in that** it is carried out in order to separate carbon monoxide from hydrogen in industrial chemistry processes resulting in this mixture.

**11.** Silica modified by a compound of formula (II) suitable for the implementation of the process as defined in one of Claims 1 to 10, in which at least one of the $R_a$, $R_b$ or $R_c$ groups represents an unsubstituted phenyl radical or a substituted phenyl radical, **characterized in that** it is obtained by introduction of spacer arms, which make possible the anchoring of the cations of formula (II') to the silica gel, which are divalent radicals bonded, on the one hand, to the said compounds via one of the 2-, 3- or 4-positions of one or more of the phenyl radicals represented by at least one of the $R_a$, $R_b$ or $R_c$ groups and bonded, on the other hand, to one or more of the free silanol functional groups of a silica gel via an Si-O covalent bond.

**12.** Modified silica as defined in Claim 11, for which the divalent radical connecting the participating silicon atom at one or more Si-O covalent bonds and the 2-, 3- or 4-position of the said phenyl radical is chosen from the following divalent radicals:

$-CH=CH-$, $-NH-(CH_2)_n-$, $-O-(CH_2)_m-$ or $-NH-(C=O)-CH_2-NH-(CH_2)_p-$,

in which n, m and p, which are identical or different, represent, independently of one another, an integer greater than or equal to 0 and less than or equal to 4.

**13.** Sol-gel material derived from a compound of formula (II) suitable for the implementation of the process as defined in one of Claims 1 to 10 in which three $R_a$, $R_b$ and $R_c$ groups represent an unsubstituted phenyl radical or a substituted phenyl radical, **characterized in that** it is obtained by substituting the 2-, 3- or 4-position of the said phenyl radicals by a monovalent radical having an end group:

$-Si(OR_{14})_3$

in which $R_{14}$ represents a methyl radical, an ethyl radical or an isopropyl radical.

**14.** Material as defined in Claim 13, in which the monovalent radical having an $-Si(OR_{14})_3$ end group is chosen from the following radicals:

$-CH=CH-Si(OR_{14})_3$, $-NH-(CH_2)_n-Si(OR_{14})_3$, $-O-(CH_2)_m-Si(OR_{14})_3$ or $-NH(C=O)-CH_2-NH-(CH_2)_p-Si(OR_{14})_3$,

in which n, m and p, which are identical or different, represent, independently of one another, an integer greater than or equal to 0 and less than or equal to 4 and $R_{14}$ represents a methyl radical, an ethyl radical or an isopropyl radical.

**15.** Mesoporous silica modified by a compound of formula (II) suitable for the implementation of the process as defined in one of Claims 1 to 10 in which at least one of the $R_a$, $R_b$ or $R_c$ groups represents an unsubstituted phenyl radical or a substituted phenyl radical, **characterized in that** it is obtained by introduction of spacer arms, which make possible the anchoring of the molecules of formula (I) or of the cations of formula (II') to the mesoporous silica, which are divalent radicals bonded, on the one hand, to the said compounds via one of the 2-, 3- or 4-positions of just one or of several of the phenyl radicals represented by at least one of the $R_a$, $R_b$ or $R_c$ groups and bonded, on the other hand, to one or more of the free silanol functional groups of the mesoporous silica via an Si-O covalent bond.

**16.** Modified mesoporous silica as defined in Claim 15,
in which the divalent radical connecting the participating silicon atom at one or more Si-O covalent bonds and the 2-, 3- or 4-position of the said phenyl radical is chosen from the following divalent radicals:

$-CH=CH-$, $-NH-(CH_2)_n-$, $-O-(CH_2)_m-$ or $-NH(C=O)-CH_2-NH-(CH_2)_p-$,

in which n, m and p, which are identical or different, represent, independently of one another, an integer greater than or equal to 0 and less than or equal to 4.

**Patentansprüche**

**1.** Verfahren zur Heraustrennung von Kohlenmonoxid aus einem Gasgemisch, das dieses enthält, **dadurch gekennzeichnet, dass** das Gemisch in Kontakt gebracht wird mit entweder einer Verbindung der Formel (II) :

$G(Y)_\alpha$,

in der G ein organometallisches Kation der Formel (II') darstellt:

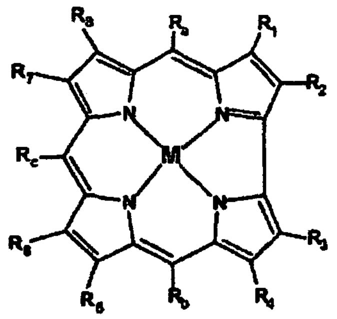

welches ausdrücklich aus einer Verbindung der Formel (I) erhalten wird:

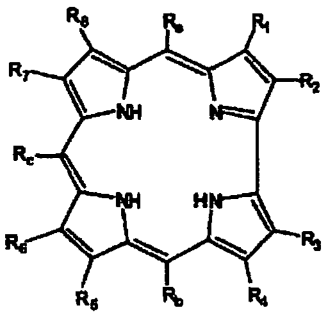

in der:

identische oder unterschiedliche $R_1$, $R_2$, $R_3$ und $R_4$ unabhängig voneinander ein Wasserstoffatom darstellen, ein lineares oder verzweigtes Alkylradikal, umfassend 1 bis 4 Kohlenstoffatome, ein nicht substituiertes oder durch eine oder mehrere Gruppen substituiertes Phenylradikal, wobei diese Gruppen identisch oder voneinander verschieden sind und unabhängig voneinander aus den Radikalen Vinyl, Hydroxy, Nitro, Amino, Cyano, Carboxy, Brom, Chlor, Fluor, Jod, Benzyloxy ausgewählt sind, wobei die linearen oder verzweigten Alkylradikale 1 bis 4 Kohlenstoffatome umfassen oder die linearen oder verzweigten Alkyloxyradikale 1 bis 4 Kohlenstoffatome umfassen, und die Alkylradikale und die Alkyloxyradikale selbst entweder nicht substituiert oder durch eine oder mehrere Gruppen Brom, Chlor, Fluor oder Jod substituiert sind;

identische oder unterschiedliche $R_5$, $R_6$, $R_7$ und $R_8$, die unabhängig voneinander ein Wasserstoffatom oder ein lineares oder verzweigtes Alkylradikal darstellen, umfassend 1 bis 4 Kohlenstoffatome; und

entweder identische oder unterschiedliche $R_a$, $R_b$ und $R_c$ unabhängig voneinander ein Wasserstoffatom, ein nicht substituiertes Phenylradikal oder ein durch eine oder mehrere identische oder unterschiedliche Gruppen substituiertes Phenylradikal, wobei diese Gruppen unabhängig voneinander unter den Radikalen Vinyl, Hydroxy, Nitro, Cyano, Carboxy, Amino, Brom, Chlor, Fluor, Jod, Benzyloxy oder Hydroxymethyl ausgewählt sind, darstellen, wobei die linearen oder verzweigten Alkylradikale 1 bis 4 Kohlenstoffatome umfassen oder die linearen oder verzweigten Alkoxyradikale 1 bis 4 Kohlenstoffatome umfassen und die Alkylradikale und die Alkoxyradikale selbst entweder nicht substituiert oder durch eine oder mehrere Gruppen Brom, Chlor, Fluor oder Jod substituiert sind,

oder die drei Gruppen $R_a$, $R_b$ und $R_c$ jeweils ein Wasserstoffatom aufweisen, wobei vorausgesetzt wird, dass in diesem Fall mindestens eines der Radikale $R_1$, $R_2$, $R_3$, $R_4$, $R_5$, $R_6$, $R_7$ oder $R_8$ kein Wasserstoffatom aufweist, wobei M in den Wasserstoffen der Pyrrol-Gruppe in der Formel (I), in der M ein Metallkation darstellt, das aus den Ionen Kobalt (Co), Rhodium (Rh), Iridium (Ir), Mangan (Mn), Eisen (Fe), Ruthenium (Ru) und Osmium (Os) ausgewählt ist, substituiert ist; Y ein organisches oder anorganisches Anion darstellt, und $\alpha$ eine Ganzzahl oder eine Dezimalzahl darstellt, so dass die Verbindung der Formel (II) elektrisch neutral ist;

oder mit einem Silizium, das durch die Verbindung der Formel (II) modifiziert ist, in der mindestens eine der Gruppen $R_a$, $R_b$ oder $R_c$ ein nicht substituiertes Phenylradikal oder ein substituiertes Phenylradikal darstellt,

das durch die Einführung von Abstandshaltern ("Spacer-Arms") erhalten wird, die die Verankerung der Kationen der Formeln (II') im Siliziumgel ermöglichen, die divalente Radikale darstellen, und die einerseits durch eine der Positionen 2, 3 oder 4 eines oder mehrerer Phenylradikale, die durch mindestens eine der Gruppen $R_a$, $R_b$ oder $R_c$ dargestellt werden, an die Verbindungen gebunden sind, und andererseits durch eine kovalente Bindung Si - O an eine oder mehrere Silanolfunktionen gebunden sind, die frei von einem Siliziumgel sind;
oder mit einem Sol-Gel-Material der Verbindung der Formel (II), in der die drei Gruppen $R_a$, $R_b$ und $R_c$ ein nicht substituiertes Phenylradikal oder ein substituiertes Phenylradikal darstellen, das erhalten wird, indem die Position 2, 3 oder 4 der Phenylradikale durch ein monovalentes Radikal ersetzt wird, welches eine Endgruppe besitzt:

$$-Si(OR_{14})_3$$

in der $R_{14}$ ein Methylradikal, ein Ethylradikal oder ein Isopropylradikal darstellt;
oder mit einem mesoporösen Silizium, das durch eine Verbindung der Formel (II) modifiziert ist, in der mindestens eine der Gruppen $R_a$, $R_b$ oder $R_c$ ein nicht substituiertes Phenylradikal oder ein substituiertes Phenylradikal darstellt, das durch die Einführung von Abstandshaltern erhalten wird, die die Verankerung der Kationen der Formeln (II') in dem mesoporösen Silizium ermöglichen, wobei diese divalente Radikale darstellen, die einerseits an die Verbindungen der Formel (II) durch eine der Positionen 2, 3 oder 4 eines oder mehrerer Phenylradikale gebunden sind, die durch mindestens eine der Gruppen $R_a$, $R_b$ oder $R_c$ dargestellt sind, und andererseits durch eine kovalente Bindung Si - O an eine oder mehrere Silanolfunktionen gebunden sind, die frei von dem mesoporösen Silizium sind.

**2.** Verfahren, wie es in Anspruch 1 definiert ist, für das in der Formel (I) eine der Gruppen $R_a$, $R_b$ oder $R_c$ ein Radikal darstellt, das aus den Radikalen 4-Brom-Phenyl, 4-Chlor-Phenyl, 4-Jod-Phenyl, 4-Nitro-Phenyl, 4-Cyano-Phenyl, 4-Carboxy-Phenyl, 4-Hydroxy-Phenyl, 4-Amino-Phenyl, 4-Hydroxymethyl-Phenyl oder 4-Vinyl-Phenyl ausgewählt ist, und die zwei anderen Gruppen aus $R_a$, $R_b$ oder $R_c$ jeweils ein Wasserstoffatom darstellen.

**3.** Verfahren, wie es in einem der Ansprüche 1 oder 2 definiert ist, für das in der Formel (I) $R_a$ und $R_b$ jeweils ein Wasserstoffatom darstellen.

**4.** Verfahren, wie es in einem der Ansprüche 1 bis 3 definiert ist, für das in der Formel (I) entweder $R_a$ und $R_c$ oder $R_b$ und $R_c$ jeweils ein Wasserstoffatom darstellen.

**5.** Verfahren, wie es in einem der Ansprüche 1 bis 4 definiert ist, für das in der Formel (I) identische oder unterschiedliche $R_1$, $R_2$, $R_3$ und $R_4$ unabhängig voneinander ein nicht substituiertes Phenylradikal oder ein Phenylradikal, das durch eine oder mehrere identische oder unterschiedliche Gruppen substituiert ist, aufweisen, wobei die Gruppen unabhängig voneinander unter den Radikalen Vinyl, Hydroxy, Nitro, Amino, Cyano, Carboxy, Brom, Chlor, Fluor, Jod, Benzyloxy ausgewählt sind, die linearen oder verzweigten Alkylradikale 1 bis 4 Kohlenstoffatome umfassen oder die linearen oder verzweigten Alkyloxyradikale 1 bis 4 Kohlenstoffatome umfassen, und wobei die Alkylradikale und die Alkyloxyradikale selbst entweder nicht substituiert sind oder durch eine oder mehrere Gruppen Brom, Chlor, Fluor oder Jod substituiert sind.

**6.** Verfahren, wie es in einem der Ansprüche 1 bis 5 definiert ist, für das in der Formel (I) identische oder unterschiedliche $R_5$, $R_6$, $R_7$ und $R_8$ unabhängig voneinander ein lineares oder verzweigtes Alkylradikal darstellen, das 1 bis 4 Kohlenstoffatome umfasst, und insbesondere ein Methylradikal oder ein Ethylradikal.

**7.** Verfahren, wie es in einem der Ansprüche 1 bis 6 definiert ist, für das in der Formel (I) $R_5$, $R_6$, $R_7$ und $R_8$ identisch sind und jeweils ein Methylradikal oder ein Ethylradikal darstellen.

**8.** Verfahren, wie es in einem der Ansprüche 1 bis 7 definiert ist, für das in der Formel (I) $R_1$, $R_2$, $R_3$ und $R_4$ identisch sind und jeweils ein Phenylradikal darstellen.

**9.** Verfahren, wie es in einem der Ansprüche 1 bis 8 definiert ist, **dadurch gekennzeichnet, dass** es durchgeführt wird, um das Vorhandensein von Kohlenmonoxid in einer gegebenen Atmosphäre und insbesondere in feuchter Luft festzustellen.

**10.** Verfahren, wie es in einem der Ansprüche 1 bis 9 definiert ist, **dadurch gekennzeichnet, dass** es durchgeführt wird, um in den Verfahren der industriellen Chemie, die zu diesem Gemisch führen, das Kohlenmonoxid von dem

Wasserstoff zu trennen.

**11.** Silizium, das durch eine Verbindung der Formel (II) modifiziert ist und das für die Durchführung des Verfahrens, wie es in einem der Ansprüche 1 bis 10 definiert ist, geeignet ist, in dem mindestens eine der Gruppen $R_a$, $R_b$ oder $R_c$ ein nicht substituiertes oder substituiertes Phenylradikal darstellt, **dadurch gekennzeichnet, dass** dieses durch die Einführung von Abstandshaltern erhalten wird, welche die Verankerung der Kationen der Formeln (II') in dem Siliziumgel ermöglichen, die divalente Radikale darstellen, die einerseits durch eine der Positionen 2, 3 oder 4 einer oder mehrerer Phenylradikale, die durch mindestens eine der Gruppen $R_a$, $R_b$ oder $R_c$ dargestellt sind, an die Verbindungen gebunden sind, und die andererseits durch eine kovalente Bindung Si - O an eine oder mehrere Silanolfunktionen gebunden sind, die frei von einem Siliziumgel sind.

**12.** Silizium, das auf eine Weise modifiziert ist, wie es in Anspruch 11 definiert ist, für das das divalente Radikal, das das Siliziumatom bindet, an einen oder mehreren kovalenten Si - O - Bindungen beteiligt ist, und die Position 2, 3 oder 4 des Phenylradikals aus den folgenden divalenten Radikalen ausgewählt ist:

$-CH=CH-$, $-NH-(CH_2)_n-$, $-O-(CH_2)_m-$ oder $-NH(C=O)-CH_2\ NH-(CH_2)_p-$,

in denen identische oder unterschiedliche n, m und p unabhängig voneinander eine Ganzzahl darstellen, die größer oder gleich 0 und kleiner oder gleich 4 ist.

**13.** Ein Sol-Gel-Material, das aus einer Verbindung der Formel (II) abgeleitet ist und das für die Durchführung des Verfahrens geeignet ist, wie es in einem der Ansprüche 1 bis 10 definiert ist, in dem die drei Gruppen $R_a$, $R_b$ und $R_c$ ein nicht substituiertes Phenylradikal oder ein substituiertes Phenylradikal darstellen, **dadurch gekennzeichnet, dass** dieses erhalten wird, indem die Position 2, 3 oder 4 der Phenylradikale durch ein monovalentes Radikal substituiert werden, welches eine Endgruppe besitzt:

$-Si(OR_{14})_3$

in der $R_{14}$ ein Methylradikal, ein Ethylradikal oder ein Isopropylradikal darstellt.

**14.** Material, wie es in Anspruch 13 definiert ist, in dem das monovalente Radikal, das eine Endgruppe $-Si(OR_{14})_3$ besitzt, aus den folgenden Radikalen ausgewählt ist:

$-CH=CH-Si(OR_{14})_3$, $-NH-(CH_2)_n-Si(OR_{14})_3$, $-O-(CH_2)_m-Si(OR_{14})_3$
oder $-NH\ (C=O)-CH_2-NH-(CH_2)_p-Si(OR_{14})_3$,
in denen identische oder unterschiedliche n, m und p unabhängig voneinander eine Ganzzahl darstellen, die größer oder gleich 0 und die kleiner oder gleich 4 ist, und $R_{14}$ ein Methylradikal, ein Ethylradikal oder ein Isopropylradikal darstellt.

**15.** Mesoporöses Silizium, das durch eine Verbindung der Formel (II) modifiziert und für die Durchführung des Verfahrens geeignet ist, wie es in einem der Ansprüche 1 bis 10 definiert ist, in denen mindestens eine der Gruppen $R_a$, $R_b$ oder $R_c$ ein nicht substituiertes Phenylradikal oder ein substituiertes Phenylradikal darstellt, **dadurch gekennzeichnet, dass** dieses durch die Einführung von Abstandshaltern erhalten wird, die die Verankerung der Moleküle der Formeln (I) oder der Kationen der Formeln (II') am mesoporösen Silizium ermöglichen, die divalente Radikale darstellen, die einerseits durch eine der Positionen 2, 3 oder 4 eines einzelnen oder mehrerer Phenylradikale, die durch mindestens eine der Gruppen $R_a$, $R_b$ oder $R_c$ dargestellt sind, an die Verbindungen gebunden sind, und die andererseits durch eine kovalente Bindung Si - O an eine oder mehrere Silanolfunktionen gebunden sind, die frei von dem mesoporösen Silizium sind.

**16.** Mesoporöses Silizium, das auf eine Weise modifiziert ist, wie es in Anspruch 15 definiert ist, in dem das divalente Radikal, das das Siliziumatom bindet, an einer oder mehreren kovalenten Bindungen Si - O beteiligt ist, und die Position 2, 3 oder 4 des Phenylradikals aus den folgenden divalenten Radikalen ausgewählt ist:

$-CH=CH-$, $-NH-(CH_2)_n-$, $-O-(CH_2)_m-$ oder $-NH(C=O)-CH_2-NH-(CH_2)_p-$,
in denen identische oder unterschiedliche n, m und p unabhängig voneinander eine Ganzzahl darstellen, die größer oder gleich 0 und kleiner oder gleich 4 ist.

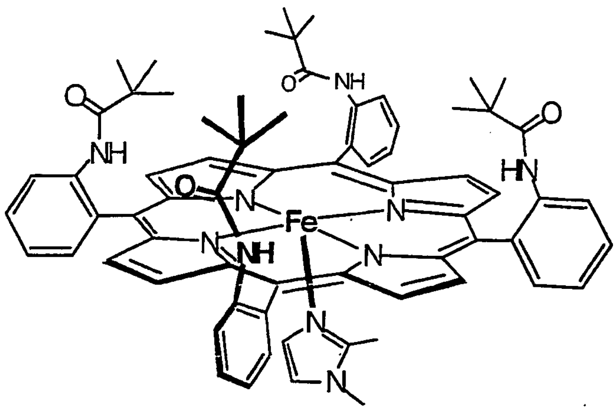

Figure 1

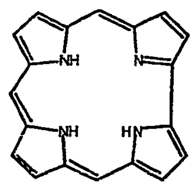

Figure 2

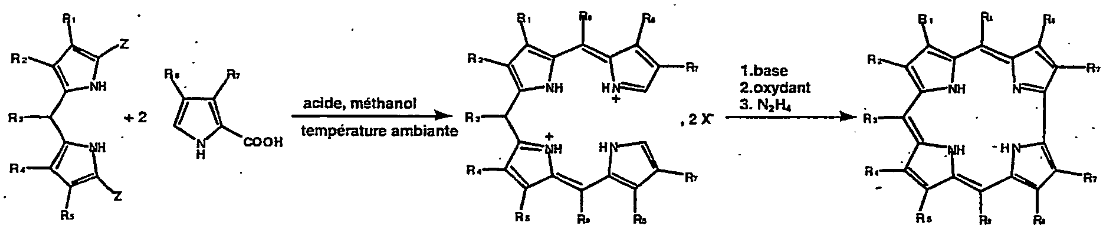


Z= CHO ou $CHR_1(OH)$

X = contre ion

Figure 3

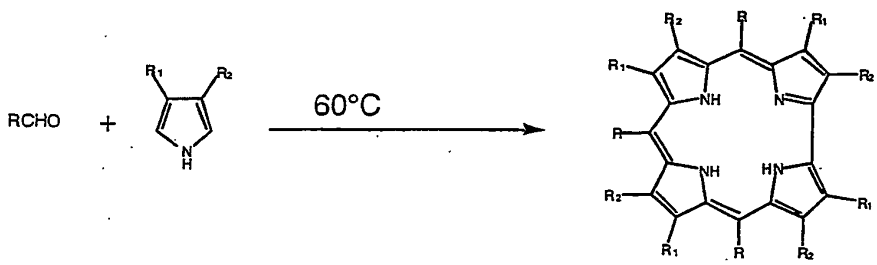


Figure 4

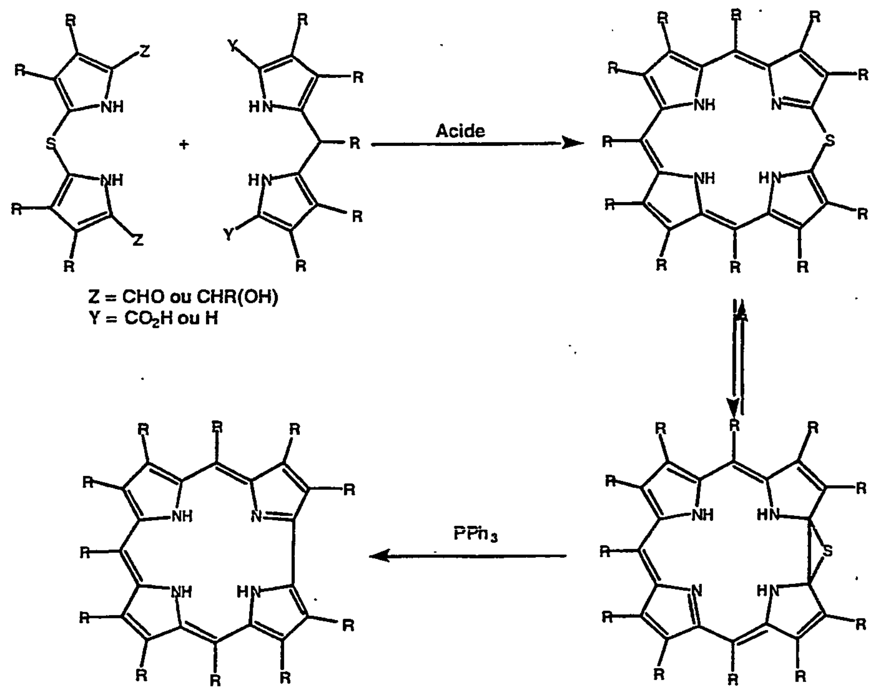


Figure 5

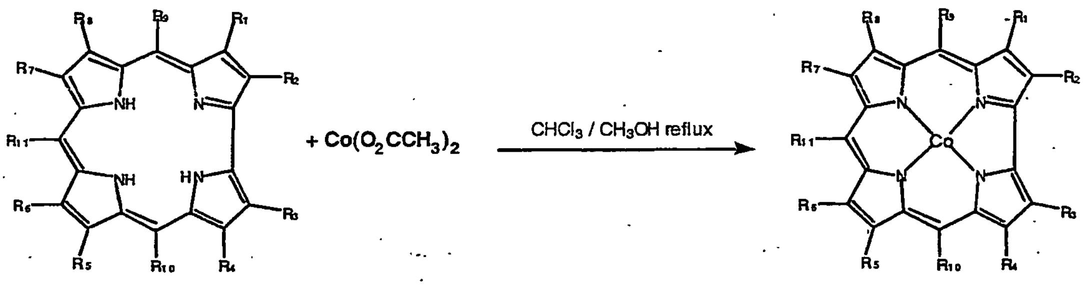


Figure 6

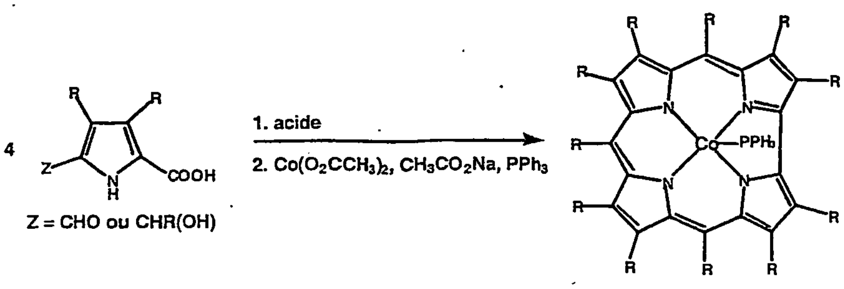


Figure 7

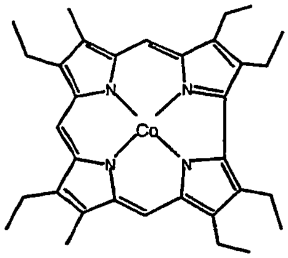

**Corrole I**

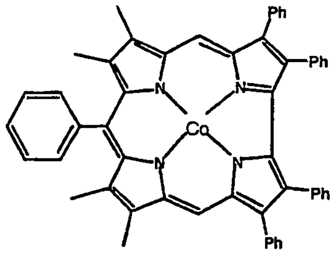

**Corrole II**

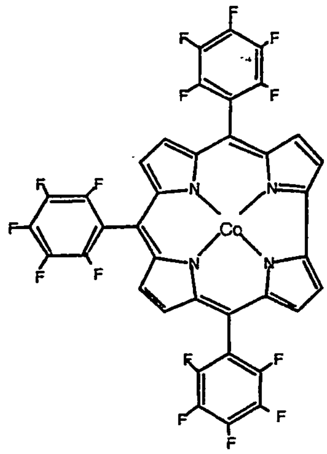

**Corrole III**

**Figure 8**

| | |
|---|---|
| Densité électronique sur le $Co^{3+}$ | Corrole I > Corrole II > Corrole III |
| Affinité attendue pour le CO | Corrole III > Corrole II > Corrole I |

**Figure 9**

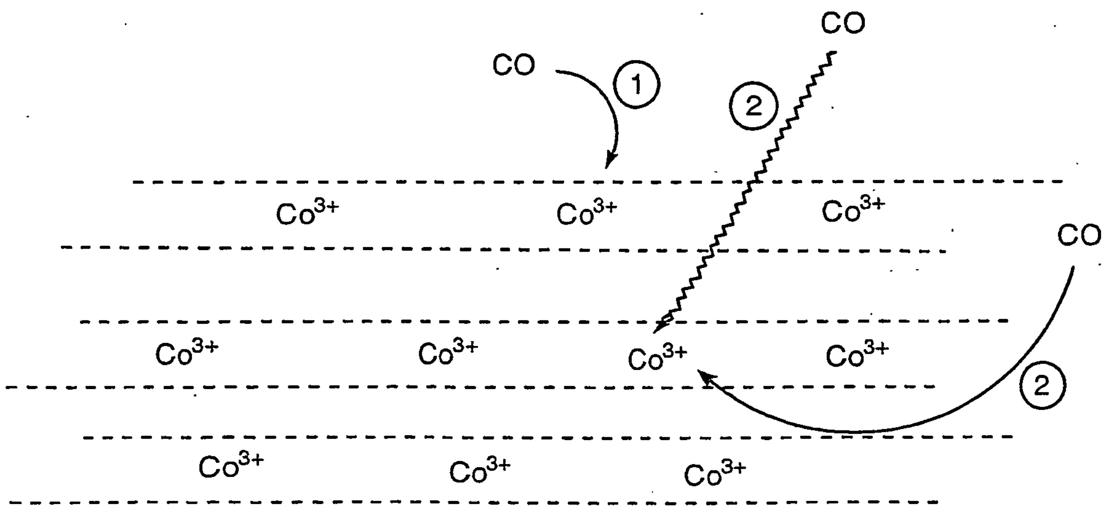


Maille Cristalline

**Figure 10**

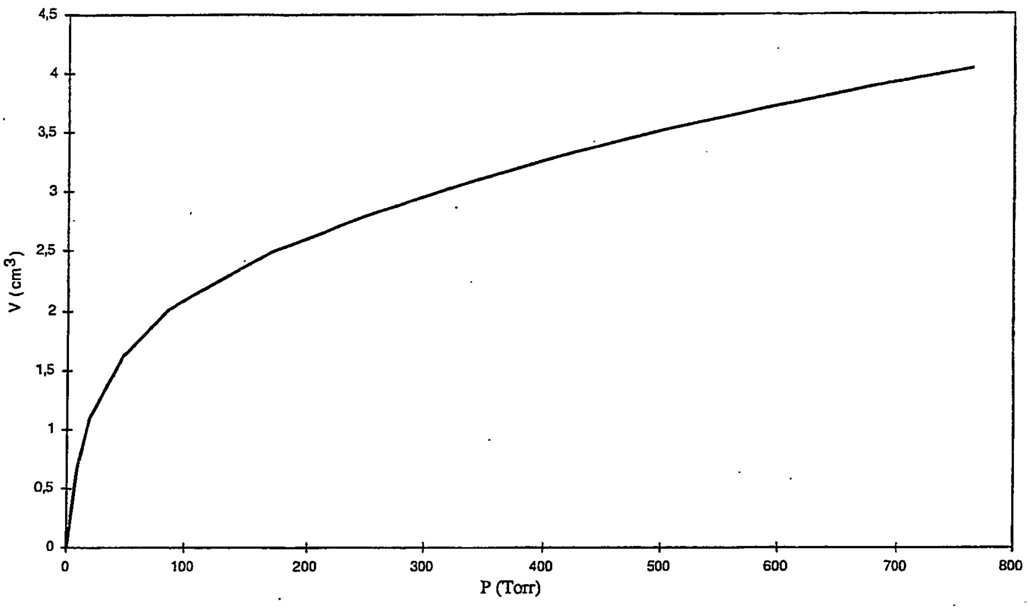


Figure 11

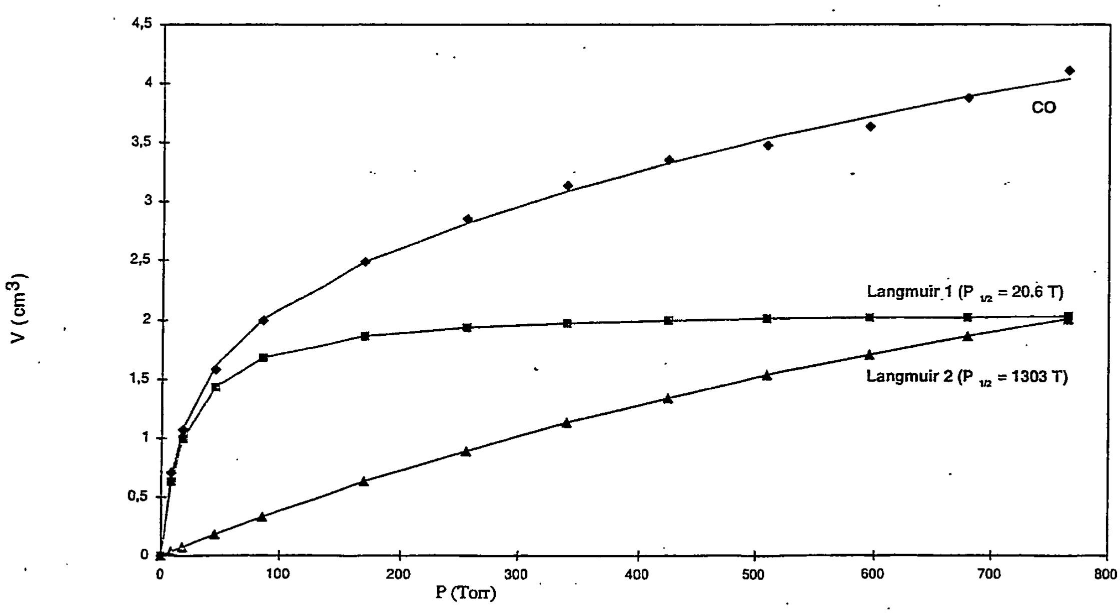


Figure 12

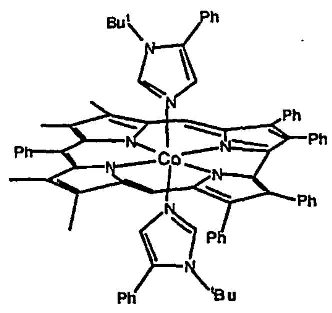

Corrole IV

Figure 13

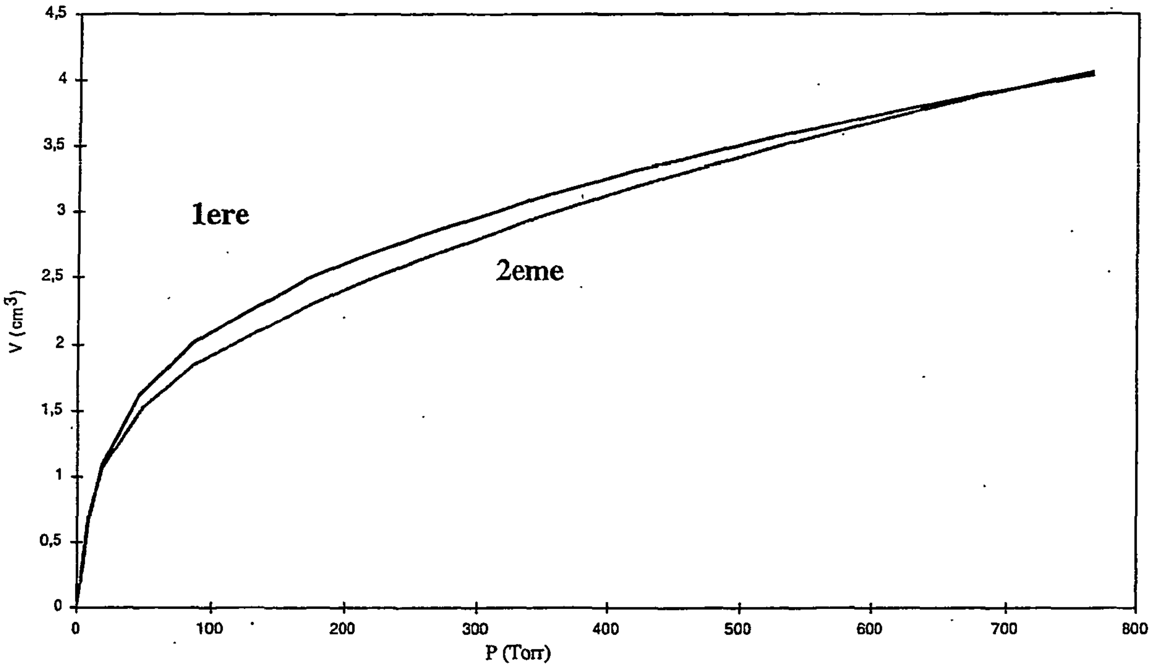


Figure 14

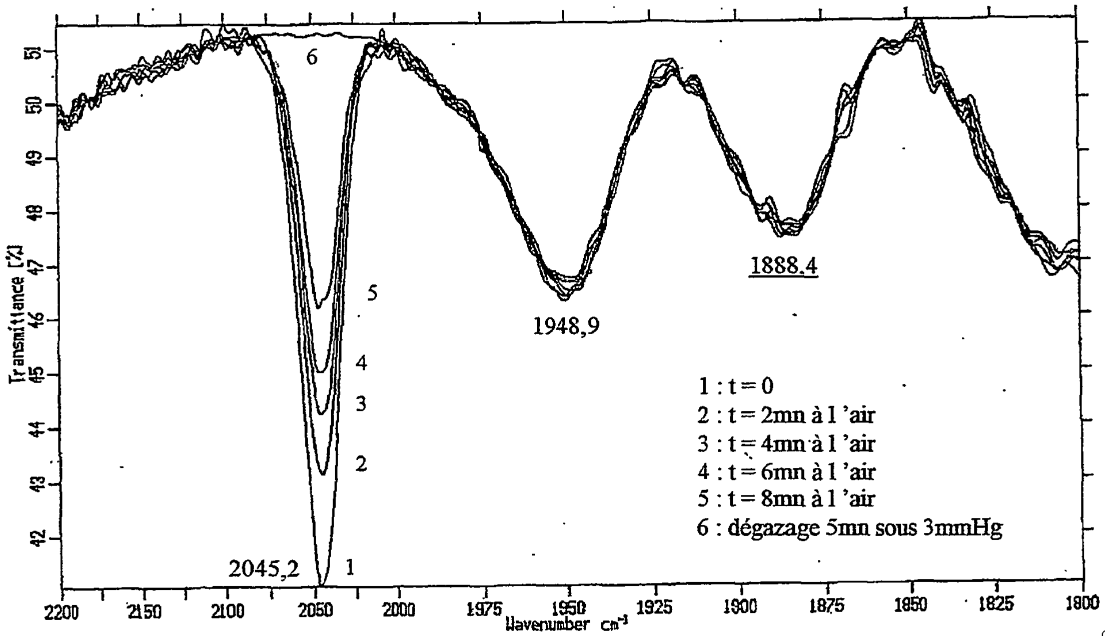


Figure 15

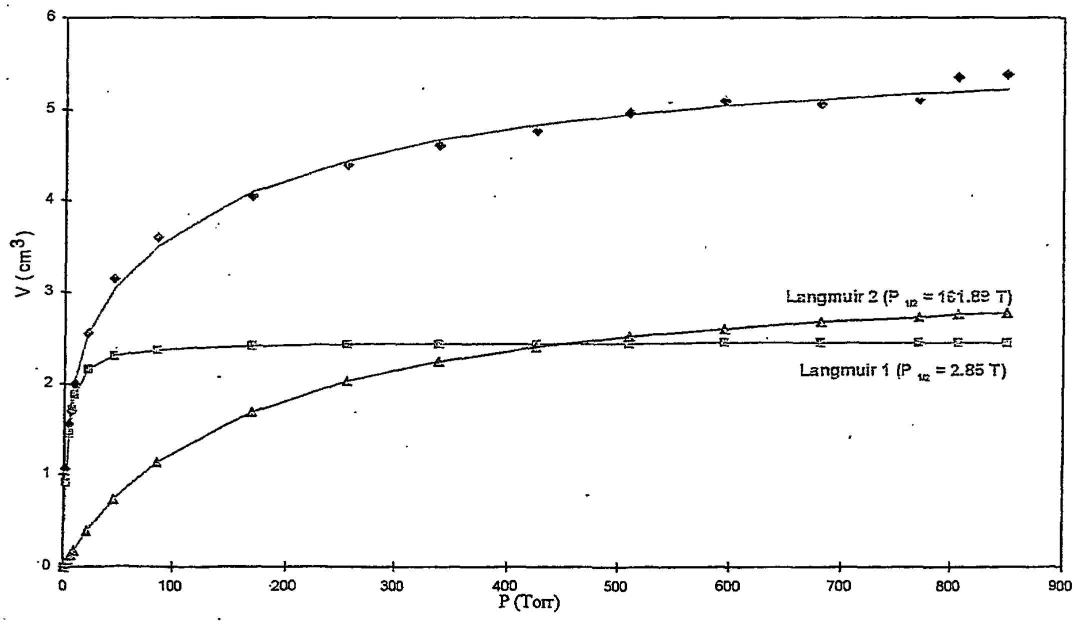


Figure 16

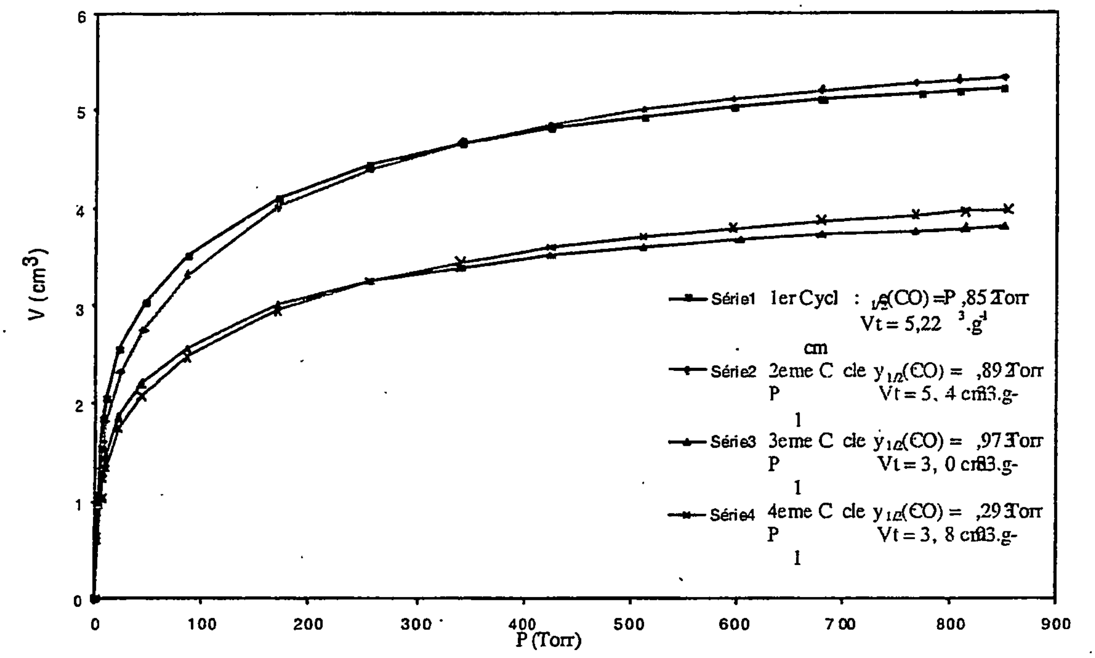


Figure 17

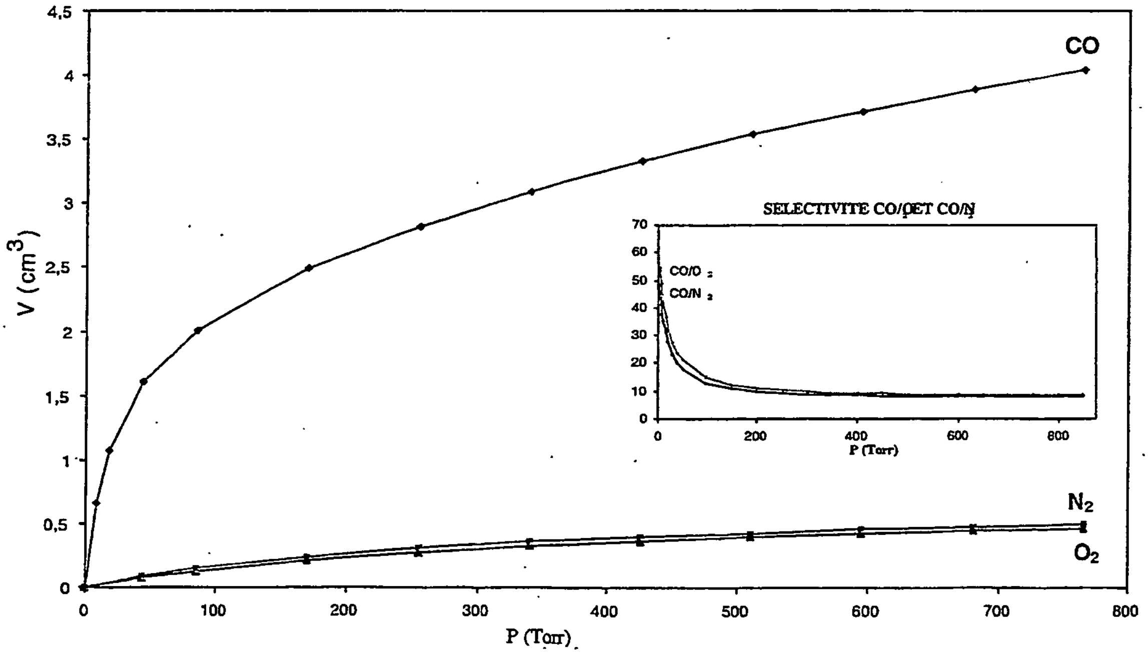


Figure 18

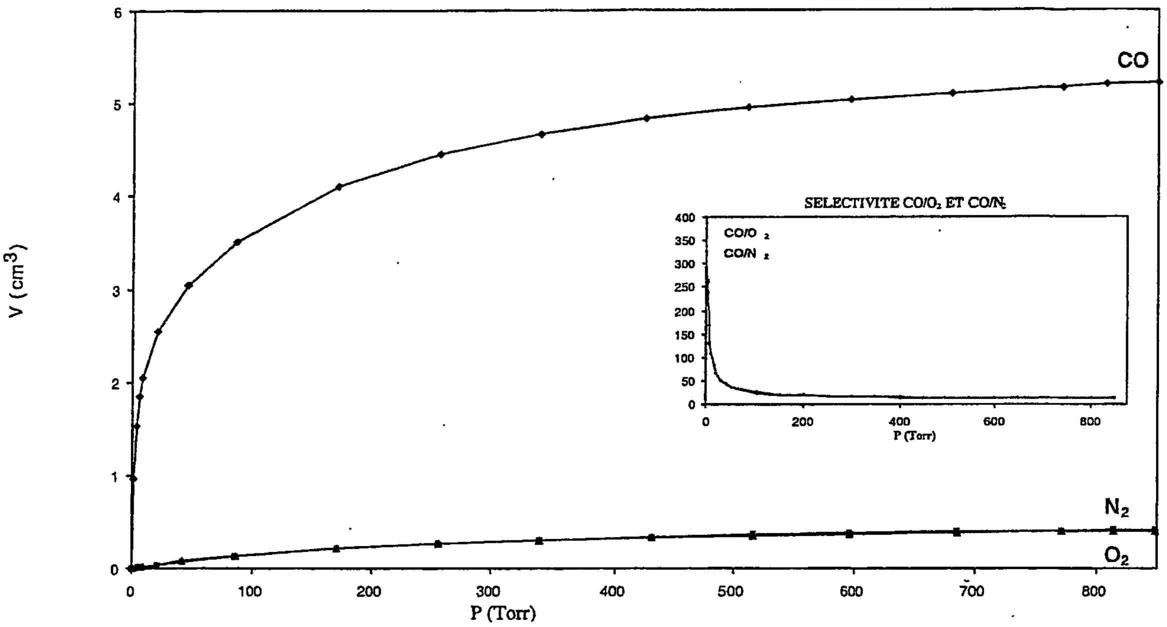


Figure 19

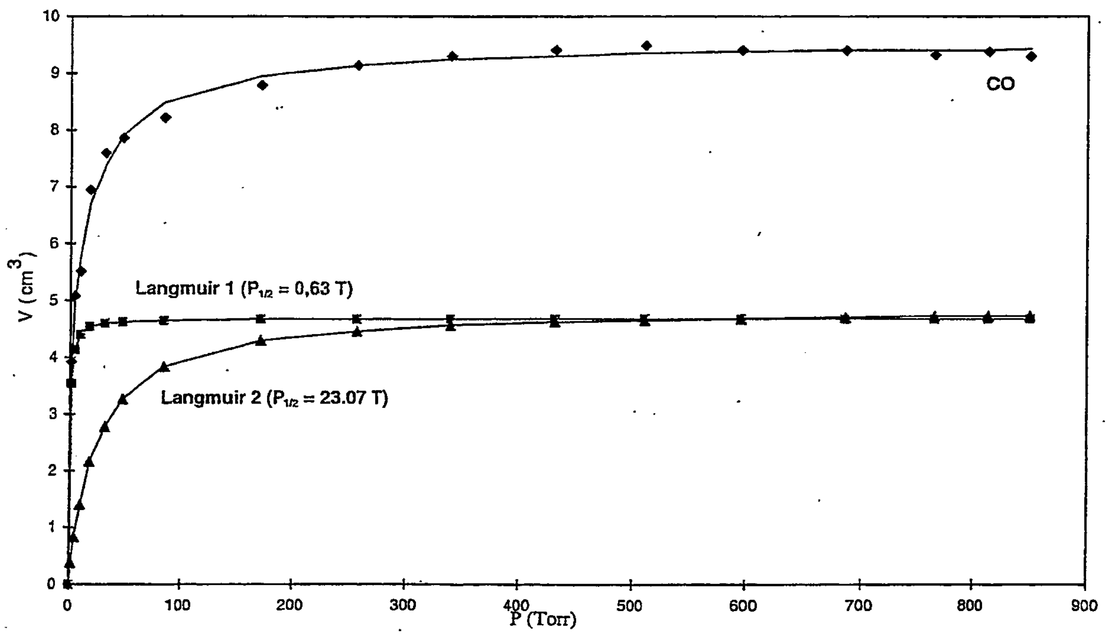


Figure 20

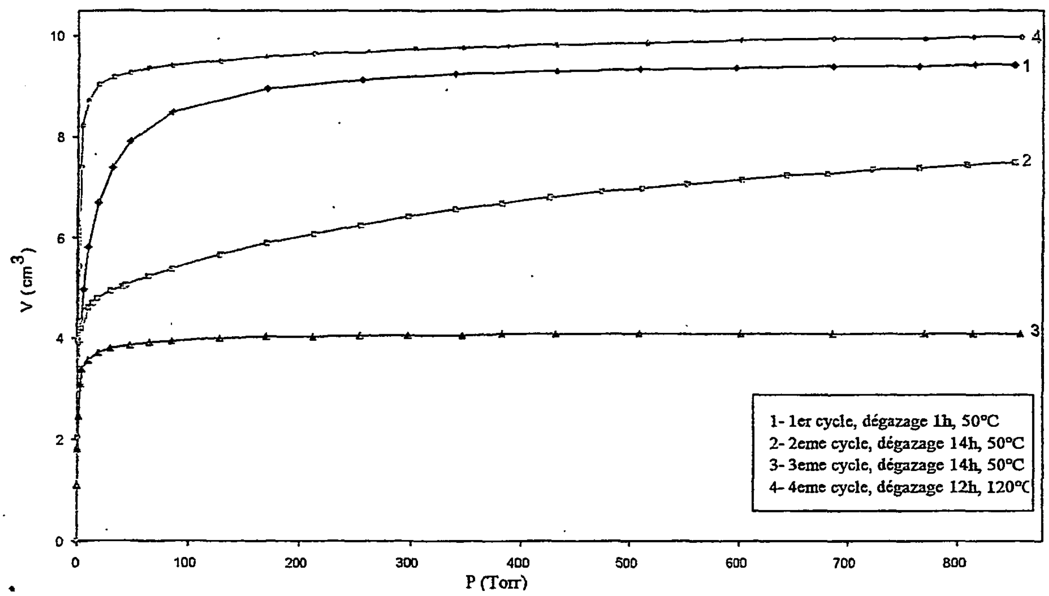


**Figure 21**

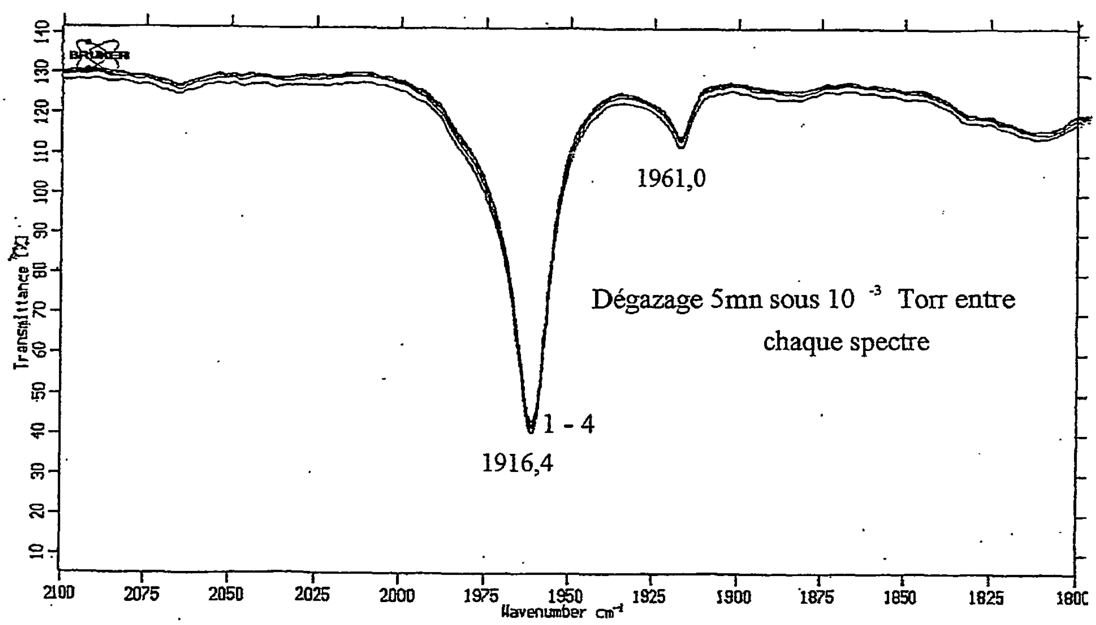


**Figure 22**

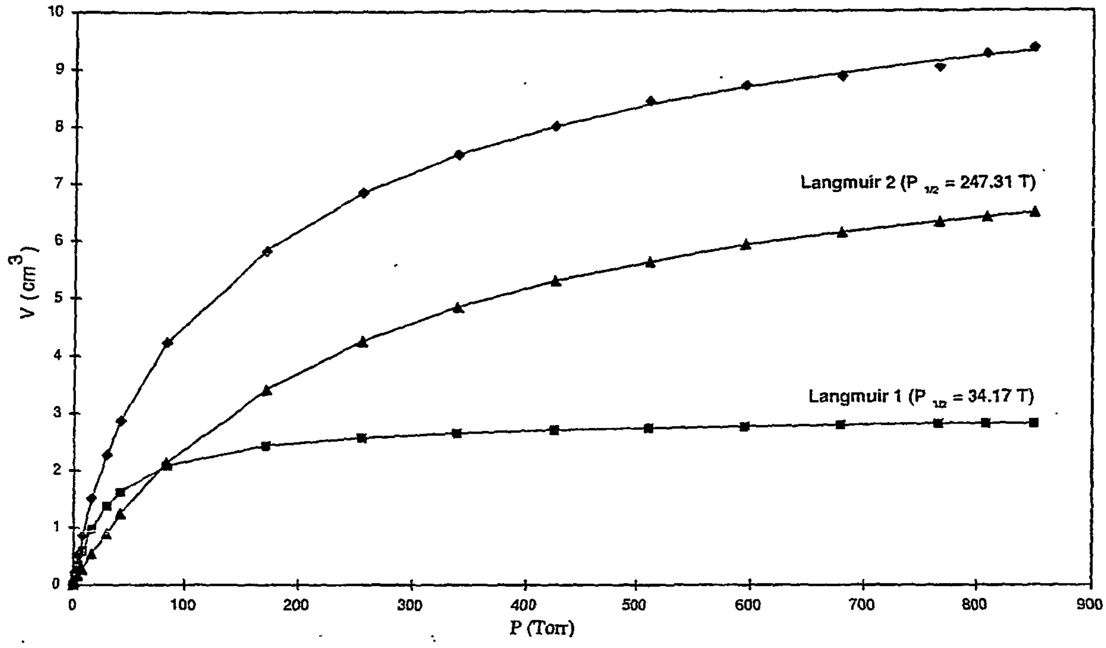


Figure 23

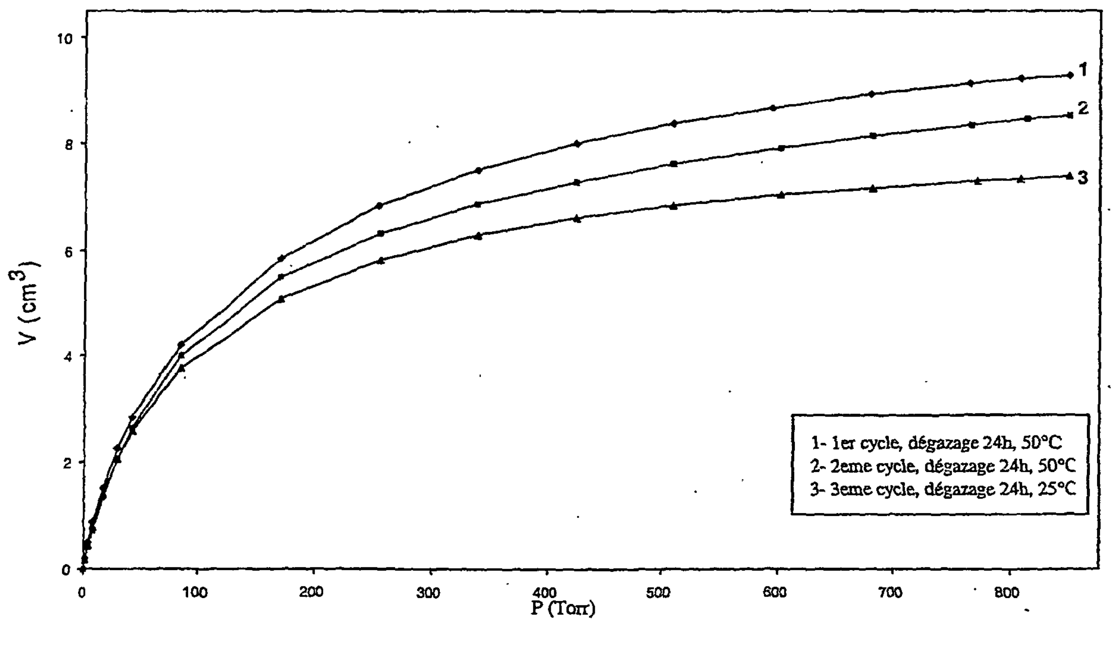


Figure 24

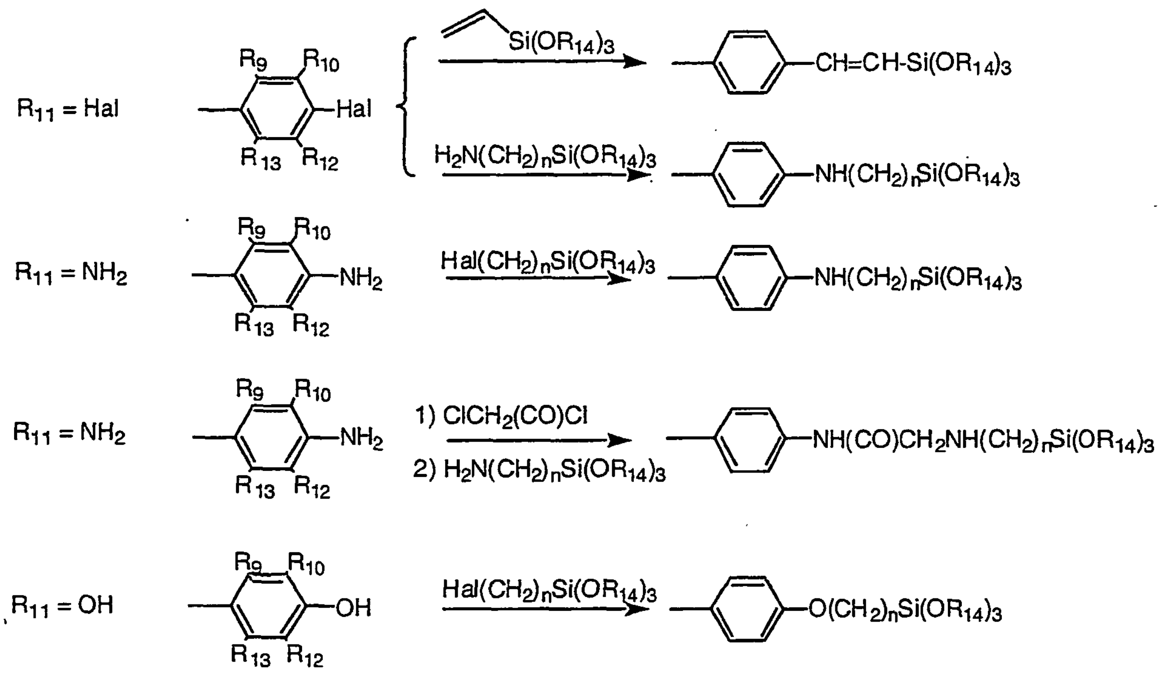


$R_{14}$ = $-CH_3$, $-C_2H_5$
n=0-4

**Schéma 1**

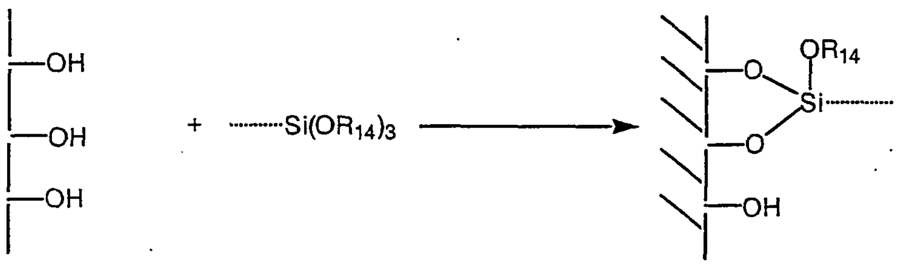

**Schéma 2**

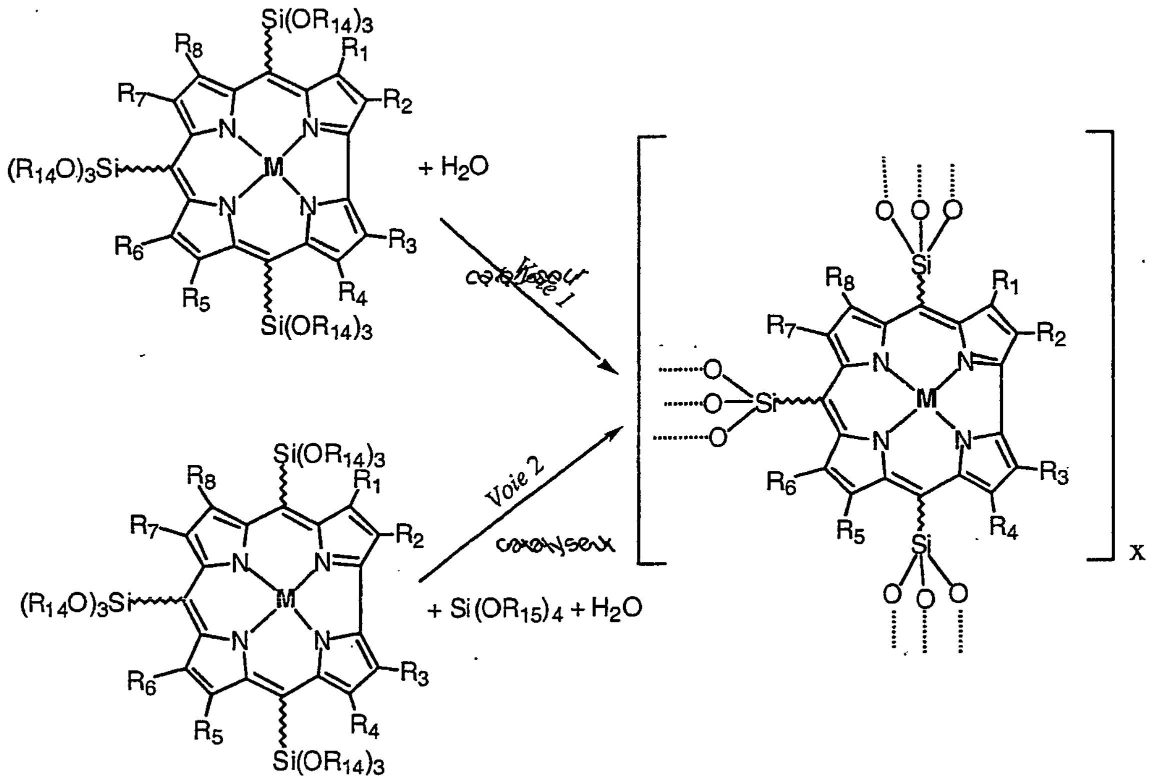


M = $H_2$, métal
$R_{14}$ = $-CH_3$, $-C_2H_5$
$R_{15}$ = $-CH_3$, $-C_2H_5$

**Schéma 3**

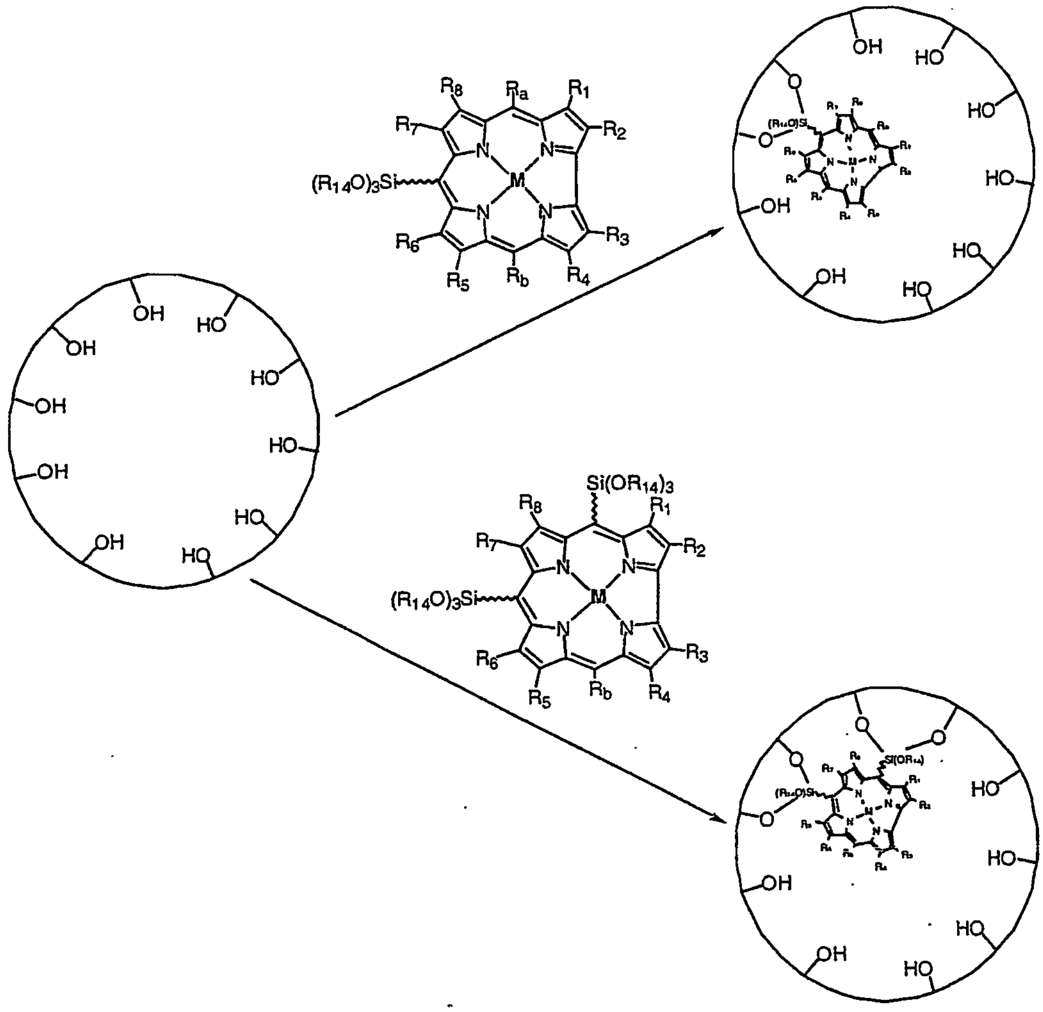

Schéma 4